# EUROPEAN PATENT APPLICATION

(11) **EP 4 722 243 A1**
(43) Date of publication of application: **08.04.2026**
(21) Application number: 24818374.1
(22) Date of filing: 16.04.2024
(51) Int. Cl.: C07K 16/24, C12N 15/13, A61K 39/395

(54) **ANTIBODY BINDING TO IL-11, ANTIGEN-BINDING FRAGMENT THEREOF, AND USES THEREOF**

(30) Priority: 05.06.2023 CN 202310652547
(71) Applicant: Beijing Dongfang Biotech Co. Ltd., Beijing 100176 (CN); Beijing Jingyitaixiang Technology Development Co. Ltd., Beijing 100176 (CN)
(72) Inventor: BAI, Yi, Beijing 100176 (CN)
(74) Representative: Müller Schupfner & Partner Patent- und Rechtsanwaltspartnerschaft mbB (Muc)
(86) International application number: PCT/CN2024/087856
(87) International publication number: WO 2024/250838

(57) **Abstract**

The present invention relates to the field of biomedicine, and specifically provides an antibody binding to IL-11 or antigen-binding fragment thereof, comprising three heavy-chain complementarity-determining regions respectively represented by HCDR1, HCDR2, and HCDR3, and three light-chain complementarity-determining regions respectively represented by LCDR1, LCDR2, and LCDR3, the antibody or antigen-binding fragment thereof being selected from A-I, A-II, A-III, or A-IV. The provided antibody or antigen-binding fragment thereof has high binding capacity to the IL-11 antigen and can block the IL-11 antigen from binding to receptors thereof, thus effectively inhibiting the pro-fibrotic effect of IL-11 and inhibiting or preventing the generation or proliferation of fibroblasts, and can be effectively used for treating or preventing human fibrotic diseases, inflammation, cancers, or autoimmune diseases.

## Description

### CROSS-REFERENCE TO THE RELATED APPLICATION

The present application claims the benefit of a Chinese Patent Application No. 202310652547.9 filed on June 5, 2023, the content of which is incorporated herein by reference in its entirety.

### TECHNICAL FIELD

The present application relates to the field of biomedical technology, and in particular to an antibody binding to IL-11, an antigen-binding fragment thereof, and uses thereof.

### BACKGROUND

Fibrosis is a repair response of tissues after injury to preserve the relative integrity of tissues and organs. Various harmful stimuli, including toxins, infectious pathogens, autoimmune reactions, and mechanical stress, can induce a fibrotic response in cells. In response to tissue injury, fibroblasts from multiple sources (including resident fibroblasts, mesenchymal cells, circulating fibroblasts, and transdifferentiation of other cell types) can initiate a wound healing response by remodeling the extracellular environment to restore tissue integrity and promote the replacement of parenchymal cells. However, persistent injury and damage may lead to dysregulation of this process, resulting in a pathological excessive deposition of extracellular matrix (Extracellular Matrix, ECM) proteins (including collagen, laminin, and fibronectin), accompanied by upregulation of fibroblast activity, a chronic inflammatory environment caused by the infiltration of macrophages and immune cells. Cytokines and growth factors are released in large quantities, including members of the transforming growth factor-β (TGF-β) family and Wingless/Int-1 (Wnt1) proteins, which are major effectors of the fibrotic process. TGF-β and Wnt1 bind to their stem cell surface receptors and initiate downstream signal transduction, leading to upregulation of expression of target gene, whose function further enhances fibroblast differentiation and the production and secretion of ECM proteins.

Worldwide, tissue fibrosis is a main cause of disability and death in many diseases. The main pathological change is the increase of fibrous connective tissue and the reduction of parenchymal cells in organs and tissues. Continuous progression can lead to organic structural destruction and hypofunction, and even failure, seriously threatening human health and life. According to relevant statistics from the United States, nearly 45% of patients who died from various diseases in that country can be attributed to fibroproliferative diseases in tissues. Currently, about 25% of the global population suffers from non-alcoholic fatty liver disease. Although non-alcoholic fatty liver disease can improve, untimely treatment will lead to its deterioration into non-alcoholic hepatitis. Non-alcoholic hepatitis is characterized by liver inflammation, hepatocyte death, and liver fibrosis, ultimately leading to cirrhosis and liver cancer. Pulmonary fibrosis is an end-stage change of a large category of lung diseases, which is characterized by fibroblast proliferation and massive extracellular matrix accumulation, accompanied by inflammatory damage and tissue structural destruction. That is, normal alveolar tissue is damaged and abnormally repaired, resulting in structural abnormalities (scar formation). The incidence and mortality of pulmonary fibrosis are increasing year by year. Pulmonary fibrosis is mostly sporadic, with an incidence of about 3/100,000 to 5/100,000, accounting for about 65% of all interstitial lung diseases. The average survival time after diagnosis is only 2.8 years, and the mortality rate is higher than that of most tumors, making it known as a "tumor-like disease". Therefore, the development of drugs for treating fibrotic diseases is particularly urgent.

Mature IL-11 (interleukin-11) is a polypeptide with 178 amino acids and a molecular weight of about 23 KD. Studies on the three-dimensional structure of IL-11 indicate that it is a four-helix bundle structure composed of four α-helices and loops connecting the α-helices. IL-11 is a pleiotropic cytokine and a member of the IL-6 cytokine family, sharing the same signal-transducing receptor subunit GP130. This family plays a crucial role in the occurrence, development, and metastasis of tumors. The production of IL-11 is induced by known major pro-fibrotic factors, including TGFβ, FGF, PDGF, CTGF, or IL-13, and local overexpression of IL-11 leads to local tissue fibrosis. The activation of the IL-11 signaling pathway depends on the binding of IL-11 to cell surface receptors. The IL-11 receptor is composed of two glycoprotein chains, IL-11Rα and GP130. IL-11Rα has the ability to bind ligands. IL-11 first binds to IL-11Rα with low affinity to form an IL-11/IL-11Rα heterodimer. The heterodimer then binds to GP130 with high affinity to form a heterotrimeric protein. The IL-11/IL-11Rα/GP130 heterotrimer homodimerizes to form a hexamer, thereby phosphorylating and activating downstream STAT signaling pathways or MAPK cascade reactions. Ultimately, IL-11 transmits signals into the cell through the GP130 signal-transducing chain, enabling the cell to receive signals for proliferation and activation. Studies have found that blocking this signaling pathway can be an effective treatment for various tumors, chronic fibrosis, and inflammatory diseases. Therefore, the development of an antibody binding to IL-11 and its antigen-binding fragment has important clinical significance.

### SUMMARY

To meet the needs of patients with fibrotic diseases domestically and internationally, the present application has screened and obtained an antibody and antigen-binding fragment thereof that can specifically bind to IL-11 and block the binding of IL-11 to its receptor.

The specific technical solutions of the present application are as follows:
The present application provides an antibody binding to IL-11 or an antigen-binding fragment thereof, comprising three heavy chain complementarity determining regions represented by HCDR1, HCDR2 and HCDR3, respectively, and three light chain complementarity determining regions represented by LCDR1, LCDR2 and LCDR3, respectively. The antibody or the antigen-binding fragment thereof is selected from any one of the following:
A-I: an amino acid sequence of the heavy chain complementarity determining region HCDR1 is as set forth in SEQ ID NO: 1, an amino acid sequence of the heavy chain complementarity determining region HCDR2 is as set forth in SEQ ID NO: 2, an amino acid sequence of the heavy chain complementarity determining region HCDR3 is as set forth in SEQ ID NO: 3, an amino acid sequence of the light chain complementarity determining region LCDR1 is as set forth in SEQ ID NO: 4, an amino acid sequence of the light chain complementarity determining region LCDR2 is as set forth in SEQ ID NO: 5, and an amino acid sequence of the light chain complementarity determining region LCDR3 is as set forth in SEQ ID NO: 6;
A-II: an amino acid sequence of the heavy chain complementarity determining region HCDR1 is as set forth in SEQ ID NO: 1, an amino acid sequence of the heavy chain complementarity determining region HCDR2 is as set forth in SEQ ID NO: 2, an amino acid sequence of the heavy chain complementarity determining region HCDR3 is as set forth in SEQ ID NO: 3, an amino acid sequence of the light chain complementarity determining region LCDR1 is as set forth in SEQ ID NO: 4, an amino acid sequence of the light chain complementarity determining region LCDR2 is as set forth in SEQ ID NO: 5, and an amino acid sequence of the light chain complementarity determining region LCDR3 is as set forth in SEQ ID NO: 7;
A-III: an amino acid sequence of the heavy chain complementarity determining region HCDR1 is as set forth in SEQ ID NO: 8, an amino acid sequence of the heavy chain complementarity determining region HCDR2 is as set forth in SEQ ID NO: 9, an amino acid sequence of the heavy chain complementarity determining region HCDR3 is as set forth in SEQ ID NO: 10, an amino acid sequence of the light chain complementarity determining region LCDR1 is as set forth in SEQ ID NO: 11, an amino acid sequence of the light chain complementarity determining region LCDR2 is as set forth in SEQ ID NO: 12, and an amino acid sequence of the light chain complementarity determining region LCDR3 is as set forth in SEQ ID NO: 13; and
A-IV: an amino acid sequence of the heavy chain complementarity determining region HCDR1 is as set forth in SEQ ID NO: 1, an amino acid sequence of the heavy chain complementarity determining region HCDR2 is as set forth in SEQ ID NO: 14, an amino acid sequence of the heavy chain complementarity determining region HCDR3 is as set forth in SEQ ID NO: 15, an amino acid sequence of the light chain complementarity determining region LCDR1 is as set forth in SEQ ID NO: 4, an amino acid sequence of the light chain complementarity determining region LCDR2 is as set forth in SEQ ID NO: 5, and an amino acid sequence of the light chain complementarity determining region LCDR3 is as set forth in SEQ ID NO: 6.

The above antibody or the antigen-binding fragments thereof provided by the present application can all specifically bind to IL-11, block the binding of the IL-11 antigen to its receptor, and inhibit IL-11-mediated signal transduction. The above antibody provided by the present application has good biological activity.

The present application further comprises a heavy chain variable region and a light chain variable region, wherein the antibody or the antigen-binding fragment thereof is selected from any one of the following:
MA-I: an amino acid sequence of the heavy chain variable region is as set forth in SEQ ID NO: 16, and an amino acid sequence of the light chain variable region is as set forth in SEQ ID NO: 17;
MA-II: an amino acid sequence of the heavy chain variable region is as set forth in SEQ ID NO: 16, and an amino acid sequence of the light chain variable region is as set forth in SEQ ID NO: 18;
MA-III: an amino acid sequence of the heavy chain variable region is as set forth in SEQ ID NO: 19, and an amino acid sequence of the light chain variable region is as set forth in SEQ ID NO: 20; and
MA-IV: an amino acid sequence of the heavy chain variable region is as set forth in SEQ ID NO: 21, and an amino acid sequence of the light chain variable region is as set forth in SEQ ID NO: 17.

The present application further comprises a heavy chain constant region and a light chain constant region, wherein an amino acid sequence of the heavy chain constant region is as set forth in one of SEQ ID NO: 23, SEQ ID NO: 24, SEQ ID NO: 25, or SEQ ID NO: 26; and an amino acid sequence of the light chain constant region is as set forth in SEQ ID NO: 22.

Furthermore, the antibody or the antigen-binding fragment thereof is a chimeric antibody molecule, and the chimeric antibody molecule further comprises a human antibody constant region.

The present application further comprises a heavy chain variable region and a light chain variable region, wherein the antibody or the antigen-binding fragment thereof is selected from any one of the following:
HA-I-A: an amino acid sequence of the heavy chain variable region is as set forth in SEQ ID NO: 31, and an amino acid sequence of the light chain variable region is as set forth in SEQ ID NO: 32;
HA-I-B: an amino acid sequence of the heavy chain variable region is as set forth in SEQ ID NO: 33, and an amino acid sequence of the light chain variable region is as set forth in SEQ ID NO: 34;
HA-I-C: an amino acid sequence of the heavy chain variable region is as set forth in SEQ ID NO: 31, and an amino acid sequence of the light chain variable region is as set forth in SEQ ID NO: 35;
HA-I-D: an amino acid sequence of the heavy chain variable region is as set forth in SEQ ID NO: 36, and an amino acid sequence of the light chain variable region is as set forth in SEQ ID NO: 37;
HA-II-A: an amino acid sequence of the heavy chain variable region is as set forth in SEQ ID NO: 31, and an amino acid sequence of the light chain variable region is as set forth in SEQ ID NO: 38;
HA-II-B: an amino acid sequence of the heavy chain variable region is as set forth in SEQ ID NO: 31, and an amino acid sequence of the light chain variable region is as set forth in SEQ ID NO: 39;
HA-II-C: an amino acid sequence of the heavy chain variable region is as set forth in SEQ ID NO: 33, and an amino acid sequence of the light chain variable region is as set forth in SEQ ID NO: 39; and
HA-II-D: an amino acid sequence of the heavy chain variable region is as set forth in SEQ ID NO: 36, and an amino acid sequence of the light chain variable region is as set forth in SEQ ID NO: 40.

The antibody or the antigen-binding fragment thereof of the present application further comprises a human antibody constant region.

Furthermore, the human antibody constant region comprises a human antibody heavy chain constant region and a human antibody light chain constant region, wherein an amino acid sequence of the human antibody heavy chain constant region is as set forth in one of SEQ ID NO: 27, SEQ ID NO: 28, or SEQ ID NO: 29; an amino acid sequence of the human antibody light chain constant region is as set forth in SEQ ID NO: 30.

Furthermore, the antibody or the antigen-binding fragment thereof is one of Fab, F(ab')2, Fv or ScFv, or a combination thereof.

The present application also provides a nucleic acid molecule encoding the antibody binding to IL-11 or the antigen-binding fragment thereof.

The present application also provides a recombinant DNA expression vector comprising the nucleic acid molecule.

The present application also provides a host cell transfected with the recombinant DNA expression vector, wherein the host cell is a prokaryotic cell, a yeast cell, an insect cell, or a mammalian cell;
preferably, the host cell is a mammalian cell, and the mammalian cell is a HEK293 cell, a CHO cell, or an NS0 cell.

The present application also provides a drug comprising the antibody binding to IL-11 or the antigen-binding fragment thereof.

The present application also provides a detection reagent for detecting the antibody binding to IL-11 or the antigen-binding fragment thereof.

The present application also provides a combined drug composition comprising the antibody binding to IL-11 or the antigen-binding fragment thereof, and further comprising a second composition, wherein the second composition is selected from the group consisting of an antibody binding to PD-1, PD-L1, VEGF, VEGFR, TNF-α, or TSLP and an antigen-binding fragment thereof.

The present application further provides use of the antibody binding to IL-11 or the antigen-binding fragment thereof in the manufacture of a medicament for treating or preventing a fibrotic disease, inflammation, cancer, or an autoimmune disease in humans.

Preferably, the fibrotic disease is selected from the group consisting of fibrosis of heart, liver, kidney, lung, gallbladder, bladder, stomach, bone marrow, penis, breast, blood vessel, eye, pancreas, spleen, brain, intestine, muscle, and skin.

Preferably, the inflammation is selected from the group consisting of hepatitis, myocarditis, nephritis, pneumonia, cholecystitis, cystitis, gastritis, osteomyelitis, prostatitis, mastitis, pancreatitis, enteritis, arthritis, polymyositis, dermatomyositis, and dermatitis.

Preferably, the cancer is selected from the group consisting of leukemia, lung cancer, gastric cancer, esophageal cancer, ovarian cancer, head and neck cancer, melanoma, renal cancer, breast cancer, colorectal cancer, liver cancer, pancreatic cancer, and bladder cancer.

Preferably, the autoimmune disease is selected from the group consisting of psoriasis, Crohn's disease, primary biliary cirrhosis, systemic lupus erythematosus, and multiple sclerosis.

The beneficial effects of the present application are as follows: the antibody or the antigen-binding fragment thereof provided by the present application has high binding affinity to the IL-11 antigen and can block the binding of the IL-11 antigen to its receptor, thereby effectively inhibiting the pro-fibrotic effect of IL-11, and suppressing or preventing the generation or proliferation of fibroblasts. It can be effectively used for treating or preventing a fibrotic disease, inflammation, cancer, or an autoimmune disease in humans. Wherein the fibrotic disease includes but is not limited to fibrosis of heart, liver, kidney, lung, gallbladder, bladder, stomach, bone marrow, penis, breast, blood vessel, eye, pancreas, spleen, brain, intestine, muscle, or skin; the inflammation includes but is not limited to hepatitis, myocarditis, nephritis, pneumonia, cholecystitis, cystitis, gastritis, osteomyelitis, prostatitis, mastitis, pancreatitis, enteritis, arthritis, polymyositis, dermatomyositis, or dermatitis; the cancer includes but is not limited to leukemia, lung cancer, gastric cancer, esophageal cancer, ovarian cancer, head and neck cancer, melanoma, renal cancer, breast cancer, colorectal cancer, liver cancer, pancreatic cancer, or bladder cancer; the autoimmune disease includes but is not limited to psoriasis, Crohn's disease, primary biliary cirrhosis, systemic lupus erythematosus, or multiple sclerosis.

### BRIEF DESCRIPTION OF THE DRAWINGS

FIG. 1 is a plasmid profile of pScFv-Disb-HS vector in Example 2 of the present application;
FIG. 2 is a comparison diagram of the affinity of gradient-diluted ELISA for anti-IL-11 phage monoclonal antibodies in Example 3 of the present application;
FIG. 3 is a profile of the vector pTSE in Example 5 of the present application;
FIG. 4 is a denaturing polyacrylamide gel electrophoresis image of the murine antibody molecule in Example 5 of the present application;
FIG. 5 is a comparison diagram of the binding ability of the murine antibody molecule to IL-11 in Example 6 of the present application;
FIG. 6 is a comparison diagram of a competitive inhibition experiment between the murine antibody and the IL-11 receptor protein IL-11RA in Example 7 of the present application;
FIG. 7 is a comparison diagram of the inhibition of IL-11 binding to the receptor IL-11RA on the surface of BaF/3-IL-11RA cells by the murine antibody in Example 8 of the present application;
FIG. 8 is a comparison diagram of the inhibition of TIMP-1 secretion by embryonic lung fibroblast MRC-5 cells by the murine antibody in Example 9 of the present application;
FIG. 9 is a denaturing polyacrylamide gel electrophoresis image of the humanized antibody molecule in Example 14 of the present application;
FIG. 10 is a comparison diagram of the binding ability of the humanized antibody molecule to IL-11 in Example 18 of the present application;
FIG. 11 is a comparison diagram of the inhibition of IL-11 binding to the receptor IL-11RA on the surface of BaF/3-IL-11RA cells by the humanized antibody molecule in Example 19 of the present application;
FIG. 12 is a comparison diagram of the inhibition of IL-11 binding to the receptor GP130 on the surface of BaF/3-GP130 cells by the humanized antibody molecule in Example 20 of the present application;
FIG. 13 is a comparison diagram of the biological activity detection (reporter gene) of the humanized antibody molecule in Example 21 of the present application;
FIG. 14 is a comparison diagram of the inhibition of TIMP-1 secretion by embryonic lung fibroblast MRC-5 cells by the humanized antibody molecule in Example 22 of the present application;
FIG. 15 is a comparison diagram of a cross-binding experiment of the humanized antibody molecule with IL-11 from different species in Example 23 of the present application;
FIG. 16 is a bar chart of the change in the lung-to-body weight ratio in the mouse pulmonary fibrosis model in Example 24 of the present application;
FIG. 17 shows images of hematoxylin and eosin (HE) staining and Masson's staining of lung tissue sections in the mouse pulmonary fibrosis model in Example 24 of the present application;
FIG. 18 is a bar chart of the change in the heart-to-body weight ratio in the mouse cardiac fibrosis model in Example 25 of the present application;
FIG. 19 shows images of hematoxylin and eosin (HE) staining and Masson's staining of heart tissue sections in the mouse cardiac fibrosis model in Example 25 of the present application;
FIG. 20 is a bar chart of the urine protein content in the kidneys in the mouse renal fibrosis model in Example 26 of the present application;
FIG. 21 shows images of hematoxylin and eosin (HE) staining and Masson's staining of kidney tissue sections in the mouse renal fibrosis model in Example 26 of the present application;
FIG. 22 is a bar chart of the change in liver weight in the mouse hepatic fibrosis model in Example 27 of the present application;
FIG. 23 is a bar chart of the changes in the levels of alanine aminotransferase (ALT) and aspartate aminotransferase (AST) in the serum of mice in the mouse hepatic fibrosis model in Example 27 of the present application;
FIG. 24 shows images of hematoxylin and eosin (HE) staining and Masson's staining of liver tissue sections in the mouse hepatic fibrosis model in Example 27 of the present application;
FIG. 25 is an evaluation diagram of the thermal stability of the anti-IL-11 monoclonal antibody HA-I-A in Example 28 of the present application.

### DETAILED DESCRIPTION

To facilitate a better understanding of the present application, the following explanations are provided for certain technical and scientific terms used herein prior to describing the examples:
The term "antibody" as used herein encompasses full antibodies and any antigen-binding fragments thereof. Antibodies include murine antibodies, humanized antibodies, bispecific antibodies, or chimeric antibodies. Antibodies can also be fragments such as Fab, F(ab')2, Fv, or ScFv (single-chain antibody). Antibodies can be naturally occurring or modified (e.g., mutated, deleted, substituted, etc.), as long as they exhibit binding to the relevant target molecule. "Antibody" herein includes its fragments and derivatives, including synthetic antibodies and fragments. As used herein, an antibody is a polypeptide capable of specifically binding to a relevant target molecule (i.e., the antigen for which the antibody is specific).

The terms "variable region" and "constant region" as used herein mean the sequence regions of the antibody heavy and light chains near the N-terminus are the variable region (V region), while the remaining amino acid sequences near the C-terminus, which are relatively stable, are the constant region (C region). The variable region includes 3 complementarity determining regions (CDRs) and 4 framework regions (FRs). Each light chain variable region and heavy chain variable region consists of 3 CDR regions and 4 FR regions. The 3 CDR regions of the heavy chain are represented by HCDR1, HCDR2, and HCDR3, respectively, and the 3 CDR regions of the light chain are represented by LCDR1, LCDR2, and LCDR3, respectively.

The term "murine antibody molecule" as used herein refers to an antibody obtained by immunizing mice with the human IL-11 antigen.

The term "chimeric antibody molecule" as used herein refers to an antibody formed by fusing the variable regions of a murine antibody with the constant regions of a human antibody, which can reduce the immune response induced by the murine antibody in the human body. Chimeric antibodies are produced using DNA recombination technology, where the genes for the variable regions of the light and heavy chains of a mouse monoclonal antibody are inserted into an expression vector containing the constant regions of a human antibody. Thus, in the expressed antibody molecule, the variable regions of the light and heavy chains are murine, the constant regions are human, and nearly 2/3 of the entire antibody molecule is human. Such produced antibodies reduce the immunogenicity of the murine antibody while retaining the ability of the parent antibody to specifically bind antigen.

The term "humanized antibody molecule" as used herein refers to an antibody where the CDRs of a murine monoclonal antibody are grafted into the variable regions of a human antibody, replacing the human antibody CDRs, thereby conferring the antigen-binding specificity of the murine monoclonal antibody to the human antibody while reducing its heterology.

The term "CHO cell" refers to Chinese hamster ovary cells. The term "HEK293E cell" refers to human embryonic kidney 293E cells. The term "NS0 cell" refers to mouse NS0 thymoma cells.

In this description, "IL-11" refers to IL-11 from any species and includes isoforms, fragments, variants, or homologs of IL-11 from any species. As used herein, a "fragment", "variant", or "homolog" of a protein may optionally be characterized by having a sequence identity of at least 60%, preferably one of 70%, 75%, 80%, 85%, 90%, 91%, 92%, 93%, 94%, 95%, 96%, 97%, 98%, 99%, or 100% to the amino acid sequence of a reference protein. In some embodiments, the fragment, variant, isoform, and homolog of the reference protein may be characterized as being capable of possessing the functions of the reference protein.

The present application is described in further detail below with reference to the following examples.

### Example 1

Example 1 of the present application provides an antibody binding to IL-11 or an antigen-binding fragment thereof, comprising three heavy chain complementarity determining regions represented by HCDR1, HCDR2 and HCDR3, respectively, and three light chain complementarity determining regions represented by LCDR1, LCDR2 and LCDR3, respectively. The antibody or the antigen-binding fragment thereof is selected from any one of the following, wherein the following CDRs are determined based on the Kabat numbering system.

| **Combination** | **HCDR1** | **HCDR2** | **HCDR3** | **LCDR1** | **LCDR2** | **LCDR3** |
|---|---|---|---|---|---|---|
| **A-I** | **DYYMF** | TISDGGTYTY YPDSVKG | DGGYVSSPEA MDY | RASQDISNYL N | **YTSRLHS** | **QQGNTLPPT** |
| | **(SEQ ID NO_{:} 1)** | **(SEQ ID NO_{:} 2)** | **(SEQ ID NO_{:} 3)** | **(SEQ ID NO_{:} 4)** | **(SEQ ID NO_{:} 5)** | **(SEQ ID NO_{:} 6)** |
| **A-II** | **DYYMF** | TISDGGTYTY YPDSVKG | DGGYVSSPEA MDY | RASQDISNYL N | **YTSRLHS** | **QQGNTLPWT** |
| | **(SEQ ID NO_{:} 1)** | **(SEQ ID NO_{:} 2)** | **(SEQ ID NO_{:} 3)** | **(SEQ ID NO_{:} 4)** | **(SEQ ID NO_{:} 5)** | **(SEQ ID NO_{:} 7)** |
| **A-III** | **SYWMH** | VIDPSDSYTT YNQKFKG | YGYDVNWYF DV | RSSQSIVHSN GNTYLE | **EVSNRFS** | **FQGSHVPWT** |
| | **(SEQ ID NO_{:} 8)** | **(SEQ ID NO_{:} 9)** | **(SEQ ID NO_{:} 10)** | **(SEQ ID NO_{:} 11)** | **(SEQ ID NO_{:} 12)** | **(SEQ ID NO_{:} 13)** |
| **A-IV** | **DYYMF** | TISDGGSYSY YPDSVKG | DGGYISSPEA MDY | RASQDISNYL N | **YTSRLHS** | **QQGNTLPPT** |
| | **(SEQ ID NO_{:} 1)** | **(SEQ ID NO_{:} 14)** | **(SEQ ID NO_{:} 15)** | **(SEQ ID NO_{:} 4)** | **(SEQ ID NO_{:} 5)** | **(SEQ ID NO_{:} 6)** |

### Example 2 Screening of murine antibody molecules

In the present application, mice were immunized with the IL-11 antigen (the IL-11 protein, IL-11-Fc antigen, and IL-11-mFc ligand protein used in subsequent experiments all refer to human IL-11), the immunization method was optimized, and a phage display library was constructed. The specific construction, screening, and identification of the phage display library are as follows:

### Step 1: Immunization of mice with the IL-11 antigen

1. Experimental animals: Species and strain: BALB/c, female, mice; body weight: 18 g to 20 g; provider of experimental animals: Beijing HFK Bioscience Co., Ltd, China.
2. Immunization: Mice were immunized, and the antigen for immunization was human IL-11 (the gene was synthesized by Nanjing Genscript Biotechnology Co., Ltd., China; and the vector was constructed, and the protein was expressed and purified by our company).

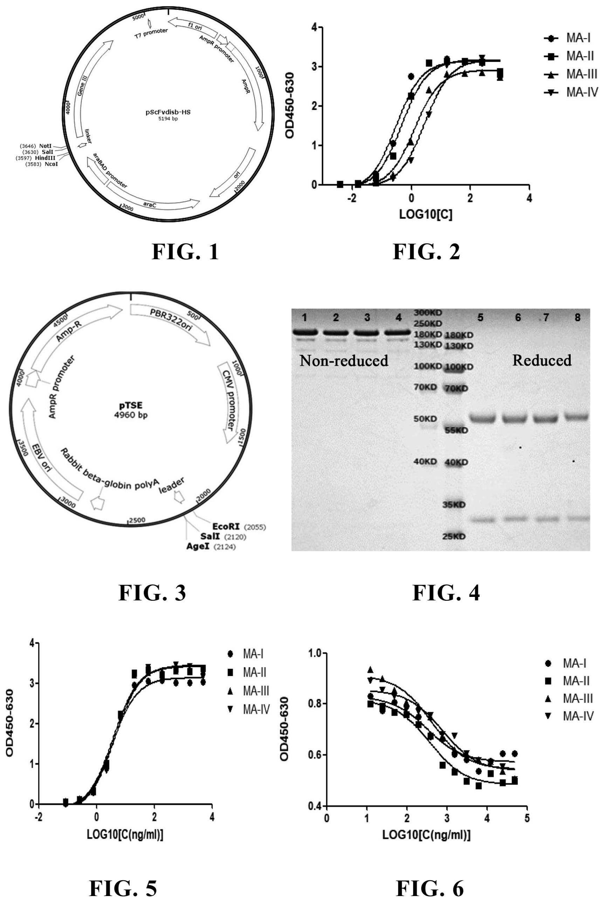

Step 2: Construction of the phage antibody library: Spleen cells from mice with high titers were taken. Total RNA was extracted from the mouse spleen cells using Trizol reagent (purchased from Ambion, Catalog No.: 15596026). cDNA was obtained by RT-PCR. Using the cDNA as a template, PCR amplification was performed with degenerate primers (for the degenerate primers used, refer to Journal of Immunological Methods 233(2000)167-177) to obtain the antibody heavy chain variable region gene library (VH) and light chain variable region gene library (VL) of the immunized mice. pScFv-Disb-HS vector was constructed by modifying pComb3 vector (purchased from Biovector Science Lab, Inc., China) through a series of gene cloning methods to make it suitable for the construction and expression of a phage single-chain antibody library. The modified vector was designated as pScFv-Disb-HS vector, and its plasmid profile is shown in FIG. 1. Based on this vector, the mouse immune phage antibody library was constructed. The light and heavy chain variable region gene libraries were separately double-digested with restriction enzymes and ligated into the similarly step-wise enzyme-digested pScFv-Disb-HS vector to construct the pScFv-Disb-HS-VH-VL gene library.

Step 3: Immunotubes were coated overnight at 4 °C with IL-11 as the antigen, with an antigen coating amount of 5 µg/500 µL/tube. The immunotubes and the immune phage antibody library were then blocked with 4% skim milk/PBST separately at room temperature for 1 hour. The blocked immune phage antibody library was added to the immunotubes for antigen-antibody binding. The input amount of phage was about 10⁹ to 10¹². After reaction at room temperature for 1 hour, unbound phages were washed away using PBST-PBS, and elution was performed using 0.1 M Glycine-HCl, pH 2.2. Finally, the eluted phage antibody solution was neutralized to about pH 7.0 using 1.5 M Tris-HCl, pH 8.8.

Step 4: 10 mL of TG1 bacterial culture grown to logarithmic phase was infected by the above neutralized phage, and let it stand in a 37 °C incubator for 30 minutes. A portion of the bacterial culture was taken for gradient dilution and plated on 2YTAG plates to calculate the phage output. The remaining bacterial culture was centrifuged, the supernatant was discarded, and the bacterial pellet was resuspended in a small amount of medium. This was then pipetted and plated on a large 2YTAG plate to prepare for the next round of screening.

Step 5: The above infected and plated bacteria were scraped from the large plate and inoculated into 2YTAG liquid medium. After shaking to logarithmic phase, M13KO7 helper phage was added for superinfection. Phages were prepared by culturing at 28 °C, 220 rpm overnight. Phages were purified by PEG/NaCl precipitation for the next round of screening. One round of phage library enrichment screening was performed in total.

Step 6: Screening of positive clones of phage single-chain antibodies against IL-11: After one round of screening, well-separated monoclonal colonies were picked and inoculated into a 96-well deep well plate containing 2YTAG liquid medium. They were cultured at 37 °C, 220 rpm until reaching logarithmic growth phase. About 10¹⁰ helper phage M13KO7 was added to each well, and static infection was performed at 37 °C for 30 minutes. After centrifugation at 4000 rpm for 15 minutes, the supernatant was discarded, and the bacterial pellet was resuspended in 2YTAK. Culture was continued at 28 °C, 220 rpm overnight. After centrifugation at 4000 rpm and 4 °C for 15 minutes, the amplified phage supernatant was pipetted for ELISA identification. Finally, four murine antibody molecules with high affinity were screened and designated as MA-I, MA-II, MA-III, and MA-IV, respectively. The obtained monoclonal antibodies were subjected to gene sequencing to confirm the correct antibody sequences. After sequencing, the sequences of the four monoclonal antibodies obtained from the above screening are as follows:

| **Murine Antibody Molecule** | **Heavy Chain Variable Region Sequence** | **Light Chain Variable Region Sequence** |
|---|---|---|
| MA-I | SEQ ID No:16 | SEQ ID No:17 |
| MA-II | SEQ ID No:16 | SEQ ID No:18 |
| MA-III | SEQ ID No:19 | SEQ ID No:20 |
| MA-IV | SEQ ID No:21 | SEQ ID No:17 |

Specifically, SEQ ID NO: 16 (the amino acid sequence of the heavy chain variable region of MA-I and MA-II): 
EVKLEESGGGLVKPGGSLKLSCAASGFTFSDYYMFWVRQTPEKRLEWVATISDGGTYT
YYPDSVKGRFTISRDNAKNNLYLQMTSLKSEDTAMYYCARDGGYVSSPEAMDYWGQGTS
VTVSS;
SEQ ID NO: 17 (the amino acid sequence of the light chain variable region of MA-I and MA-IV): 
DIVLTQSTSSLSASLGDRVTISCRASQDISNYLNWYQQKPDGTVKLLIYYTSRLIISGVPS
RFSGSGSGTDYSLTISNLEQEDIATYFCQQGNTLPPTFGGGTKLEIK;
SEQ ID NO: 18 (the amino acid sequence of the light chain variable region of MA-II): 
DIVLTQSTSSLSASLGDRVTISCRASQDISNYLNWYQQKPDGTVKLLIYYTSRLHSGVPS
RFSGSGSGTDYSLTISNLEQEDIATYFCQQGNTLPWTFGGGTKLEIK;
SEQ ID NO: 19 (the amino acid sequence of the heavy chain variable region of MA-III): 
EVKLEQSGAEVVKPGALVKMSCKASGYTFTSYWMHWVKQRPGQGLEWIGVIDPSDS
YTTYNQKFKGKATLTVDTSSSTGYMQLSSLTSEDSAVYYCSQYGYDVNWYFDVWGAGTTV
TVSS;
SEQ ID NO: 20 (the amino acid sequence of the light chain variable region of MA-III): 
DIVMTQTTLSLPVSLGDQASISCRSSQSIVHSNGNTYLEWYLQKPGQSPKLLIYEVSNR
FSGVPDRFSGSGSGTDFTLKISRVEAEDLGVYYCFQGSHVPWTFGGGTKLEIK;
SEQ ID NO: 21 (the amino acid sequence of the heavy chain variable region of MA-IV):

EVQLEESGGGLVKPGGSLKLSCVASGFTFSDYYMFWVRQTPEKRLEWVATISDGGSYS
YYPDSVKGRFTISRDNAKNNLYLQMSSLRSEDTAMYYCARDGGYISSPEAMDYWGQGTSV
TVSS.

From another perspective, the present application also relates to an antibody binding to IL-11 or an antigen-binding fragment thereof, specifically comprising a heavy chain variable region and a light chain variable region. The heavy chain variable region comprises three heavy chain complementarity determining regions represented by HCDR1, HCDR2, and HCDR3, respectively, and the light chain variable region comprises three light chain complementarity determining regions represented by LCDR1, LCDR2, and LCDR3, respectively, wherein the HCDR1, HCDR2, and HCDR3 are those of the heavy chain variable region as set forth in SEQ ID NOs: 16, 19, or 21, respectively; and the LCDR1, LCDR2, and LCDR3 are those of the light chain variable region as set forth in SEQ ID NOs: 17, 18, or 20, respectively. Those skilled in the art can determine the CDR sequences of a heavy chain variable region or light chain variable region with a known amino acid sequence based on common numbering systems (such as Kabat, AbM, Chothia, Contact, or IMGT, etc.). When the Kabat numbering system is used to determine the CDRs, the CDR sequences of the heavy chain variable region and light chain variable region are as shown in Example 1.

### Example 3 Gradient-diluted ELISA for comparing antibody affinity

The four murine antibody molecules (MA-I, MA-II, MA-III, and MA-IV) obtained in Example 2 were subjected to monoclonal phage display and purification, followed by a phage gradient-diluted ELISA experiment to identify their affinity. The specific method is as follows:
The IL-11 antigen was coated overnight at 4 °C using a pH 9.6 carbonate buffer at 100 ng/well/100 µL. After washing three times with PBST, the four phage monoclonal antibodies screened in Example 2 were each subjected to a five-fold serial dilution with PBST. 100 µL of diluted sample was added per well, and let it stand at room temperature for 1 hour. The ELISA plate was washed with PBST. HRP-anti-M13 monoclonal antibody (purchased from Bio-viewshine, Catalog No.: GE27-9421-01) diluted with 1% BSA-PBST was added to the ELISA plate, and let it stand at room temperature for 1 hour. Color development was performed for 10 minutes at room temperature using a TMB color development kit (purchased from CWBIO, Catalog No.: CW0050S), and then stopped with 2 M H₂SO₄. The plate was read at 450 nm/630 nm using a microplate reader. The corresponding EC₅₀ was calculated. The specific data are as follows:

| **Clone** | MA-I | MA-II | MA-III | MA-IV |
|---|---|---|---|---|
| EC50 | 0.2748 | 0.4436 | 1.215 | 2.732 |

Based on the above data and as shown in FIG. 2, the four different murine antibody molecules screened in Example 2 can all bind to IL-11, indicating that the monoclonal antibodies provided by the present application all possess high affinity for IL-11.

### Example 4

Example 4 of the present application further defines, on the basis of Example 2, that the antibody or the antigen-binding fragment thereof further comprises a heavy chain constant region and a light chain constant region. The amino acid sequence of the heavy chain constant region is as set forth in one of SEQ ID NO: 23, SEQ ID NO: 24, SEQ ID NO: 25, or SEQ ID NO: 26; and the amino acid sequence of the light chain constant region is as set forth in SEQ ID NO: 22. The specific sequences are as follows:
SEQ ID NO: 22 (the amino acid sequence of the light chain constant region of mouse C_{κ} type): 
ADAAPTVSIFPPSSEQLTSGGASVVCFLNNFYPKDINVKWKIDGSERQNGVLNSWTDQ
DSKDSTYSMSSTLTLTKDEYERHNSYTCEATHKTSTSPIVKSFNRNEC;
SEQ ID NO: 23 (the amino acid sequence of the heavy chain constant region of mouse IgG1 type): 
AKTTPPSVYPLAPGSAAQTNSMVTLGCLVKGYFPEPVTVTWNSGSLSSGVHTFPAVLQ
SDLYTLSSSVTVPSSTWPSETVTCNVAHPASSTKVDKKIVPRDCGCKPCICTVPEVSSVFIFPP
KPKDVLTITLTPKVTCVVVDISKDDPEVQFSWFVDDVEVHTAQTQPREEQFNSTFRSVSELPI
MHQDWLNGKEFKCRVNSAAFPAPIEKTISKTKGRPKAPQVYTIPPPKEQMAKDKVSLTCMI
TDFFPEDITVEWQWNGQPAENYKNTQPIMDTDGSYFVYSKLNVQKSNWEAGNTFTCSVLH
EGLHNHHTEKSLSHSPG;
SEQ ID NO: 24 (the amino acid sequence of the heavy chain constant region of mouse IgG2a type): 
AKTTAPSVYPLAPVCGDTTGSSVTLGCLVKGYFPEPVTLTWNSGSLSSGVHTFPAVLQS
DLYTLSSSVTVTSSTWPSQSITCNVAIIPASSTKVDKKIEPRGPTIKPCPPCKCPAPNLLGGPSV
FIFPPKIKDVLMISLSPIVTCVVVDVSEDDPDVQISWFVNNVEVIITAQTQTIIREDYNSTLRV
VSALPIQHQDWMSGKEFKCKVNNKDLPAPIERTISKPKGSVRAPQVYVLPPPEEEMTKKQV
TLTCMVTDFMPEDIYVEWTNNGKTELNYKNTEPVLDSDGSYFMYSKLRVEKKNWVERNSY
SCSVVIIEGLHNIIHTTKSFSRTPGK;
SEQ ID NO: 25 (the amino acid sequence of the heavy chain constant region of mouse IgG2b type): 
AKTTPPSVYPLAPGCGDTTGSSVTLGCLVKGYFPESVTVTWNSGSLSSSVHTFPALLQS
GLYTMSSSVTVPSSTWPSQTVTCSVAHPASSTTVDKKLEPSGPISTINPCPPCKECHKCPAPN
LEGGPSVFIFPPNIKDVLMISLTPKVTCVVVDVSEDDPDVQISWFVNNVEVHTAQTQTHRED
YNSTIRVVSTLPIQHQDWMSGKEFKCKVNNKDLPSPIERTISKIKGLVRAPQVYILPPPAEQL
SRKDVSLTCLVVGFNPGDISVEWTSNGHTEENYKDTAPVLDSDGSYFIYSKLNMKTSKWEK
TDSFSCNVRHEGLKNYYLKKTISRSPGK;
SEQ ID NO: 26 (the amino acid sequence of the heavy chain constant region of mouse IgG3 type):

ATTTAPSVYPLVPGCSDTSGSSVTLGCLVKGYFPEPVTVKWNYGALSSGVRTVSSVLQS
GFYSLSSLVTVPSSTWPSQTVICNVAHPASKTELIKRIEPRIPKPSTPPGSSCPPGNILGGPSVFI
FPPKPKDALMISLTPKVTCVVVDVSEDDPDVHVSWFVDNKEVHTAWTQPREAQYNSTFRV
VSALPIQHQDWMRGKEFKCKVNNKALPAPIERTISKPKGRAQTPQVYTIPPPREQMSKKKVS
LTCLVTNFFSEAISVEWERNGELEQDYKNTPPILDSDGTYFLYSKLTVDTDSWLQGEIFTCSV
VHEALHNHHTQKNLSRSPELELNETCAEAQDGELDGLWTTITIFISLFLLSVCYSASVTLFKV
KWIFSSVVQVKQTAIPDYRNMIGQGA.

### Example 5 Preparation of murine antibody molecules

Example 5 of the present application preferably defines, on the basis of Example 4, that the murine antibody molecule comprises the heavy chain constant region of mouse IgG1 type (its amino acid sequence is as set forth in SEQ ID NO: 23) and the light chain constant region of mouse C_{κ} type (its amino acid sequence is as set forth in SEQ ID NO: 22). The antibody preparation method is specifically as follows:
1. The encoding genes for the heavy chain VH and light chain VL of the four antibody molecules screened in Example 2 were cloned into the vector pTSE (as shown in FIG. 3) containing heavy chain and light chain constant region genes. Preferably, the heavy chain constant region is the constant region of mouse IgG1 type (the amino acid sequence is as set forth in SEQ ID NO: 23), and the light chain constant region is the murine C_{κ} chain (the amino acid sequence is as set forth in SEQ ID NO: 22). The structure of pTSE vector is shown in FIG. 3 (for the preparation process of pTSE vector, refer to paragraph [0019] on page 3 of the description of CN103525868A).
2. HEK293 cells (purchased from the Institute of Basic Medical Sciences of Chinese Academy of Medical Sciences, Catalog No.: GNHu43) were transiently transfected for antibody expression. The four monoclonal antibodies were purified using an AKTA instrument with a protein A affinity column. The protein concentration was determined using a BCA kit (purchased from Beijing Huitian Dongfang Science and Technology Co., Ltd., China, Catalog No.: BCA0020). Subsequently, the protein size was identified by SDS-PAGE. The results are shown in FIG. 4. From left to right: non-reduced MA-I, MA-II, MA-III, MA-IV, protein molecular weight marker 1, and protein molecular weight marker 2, and reduced murine anti-IL-11 monoclonal antibodies MA-I, MA-II, MA-III, and MA-IV. The molecular weight of each band is consistent with the theoretical value.

### Example 6 Binding experiment of murine antibody molecules to IL-11

The IL-11 antigen was coated overnight at 4 °C using a pH 9.6 carbonate buffer at 100 ng/well/100 µL. After washing five times with 300 µL/well of PBST, 1% BSA-PBST was added at 280 µL/well, and the plate was blocked at 37 °C for 1 hour. Differently diluted concentrations of murine antibody molecules MA-I, MA-II, MA-III, and MA-IV were added. The starting highest concentration for all four antibody molecules was 5 µg/mL. Each antibody was subjected to 5-fold serial dilution, with a total of 8 dilution gradients per antibody. The plate was incubated at 37 °C for 1 hour. After washing five times with 300 µL/well of PBST, goat anti-mouse IgG-HRP (purchased from Solarbio, Catalog No.: SE131) diluted 1:2000 with 1% BSA-PBST was added, and the plate was incubated at 37 °C for 1 hour. Color development was performed for 8 minutes at room temperature using a TMB color development kit at 100 µL/well, and then stopped with 2 M H₂SO₄. The plate was read at 450 nm/630 nm using a microplate reader, and the corresponding EC₅₀ was calculated. The specific data are as follows:

| **Clone** | MA-I | MA-II | MA-III | MA-IV |
|---|---|---|---|---|
| EC50(ng/ml) | 3.412 | 3.720 | 3.792 | 4.248 |

Based on the above data and as shown in FIG. 5, the four different screened murine antibody molecules can all bind to IL-11 with high affinity.

### Example 7 Competitive inhibition experiment of murine antibodies with IL-11 receptor protein IL-11RA

IL-11-Fc was coated overnight at 4 °C using a pH 9.6 carbonate buffer at 200 ng/well/100 µL. After washing five times with 300 µL/well of PBST, 1% BSA-PBST was added at 280 µL/well, and the plate was blocked at 37 °C for 1 hour. First, IL-11RA-Fc (IgG4 type), which was diluted to 0.5 µg/mL with 1% BSA-PBST, was added at 50 µL/well. Then, different dilution concentrations of murine antibodies MA-I, MA-II, MA-III, and MA-IV were added at 50 µL/well. The starting highest concentration for all five antibodies was 100 µg/mL. Each antibody was subjected to 2-fold serial dilution, with a total of 13 dilution gradients per antibody. The plate was incubated at 37 °C for 3 hours. After washing five times with 300 µL/well of PBST, anti-human IgG4-HRP mouse monoclonal antibody (purchased from Sigma, Catalog No.: SAB4200770) diluted 1:5000 with 2% BSA-PBST was added, and the plate was incubated at 37 °C for 1 hour. Color development was performed for 15 minutes at room temperature using a TMB color development kit at 100 µL/well, and then stopped with 2 M H₂SO₄. The plate was read at 450 nm/630 nm using a microplate reader, and the corresponding IC₅₀ was calculated. The specific data are as follows:

| **Clone** | MA-I | MA-II | MA-III | MA-IV |
|---|---|---|---|---|
| IC50(ng/ml) | 330.9 | 378.4 | 421.1 | 778.1 |

Based on the above data and as shown in FIG. 6, the four different screened murine antibodies can all compete with the receptor protein IL-11RA, indicating that they can effectively inhibit the binding of IL-11 to the receptor protein IL-11RA.

### Example 8 Inhibition of IL-11 binding to the IL-11RA receptor on the surface of BaF/3-IL-11RA cells by murine antibodies

BaF/3-IL-11RA cell strain was counted. A certain number of cells were taken, centrifuged, and resuspended in PBS buffer. The cell density was adjusted to 1E + 6 cells/mL, and the cells were added to a 96-well plate at 100 µL/well. IL-11-mFc ligand protein was diluted with PBS to a concentration of 18 µg/mL, and added at 50 µL/well to the corresponding positions of the 96-well plate containing BaF/3-IL-11RA cells. After gentle mixing, the 96-well plate was placed at 4 °C and incubated for 1 hour. The four murine antibody molecules MA-I, MA-II, MA-III, and MA-IV were diluted serially with PBS, wherein the initial concentration was 800 µg/mL, and the antibody molecules were subjected to 3-fold serial dilution for a total of 10 gradients. They were added at 50 µL/well to the corresponding positions of the 96-well plate containing the mixture of BaF/3-IL-11RA cells and IL-11-mFc ligand protein. After mixing evenly, the plate was incubated at 4 °C for 2 hours. After incubation, centrifugation was performed at 3000 rpm, the cells were washed once with PBS buffer, and the cell pellet was collected. Pre-prepared goat anti-mouse IgG Human ads-FITC antibody (purchased from SouthernBiotech, Catalog No.: 1030-02) was added to the cell pellet, and the plate was incubated at 4 °C for 30 minutes. After centrifugation at 3000 rpm, the cells were washed once with PBS buffer, resuspended in 100 µL PBS buffer, and analyzed by flow cytometry. Fluorescence signals in the FL1-A channel were collected. The dose-response curve was plotted, and the corresponding IC₅₀ was calculated. The specific data are as follows:

| **Clone** | MA-I | MA-II | MA-III | MA-IV |
|---|---|---|---|---|
| IC50 (µg/mL) | 3.297 | 11.13 | 19.05 | 59.07 |

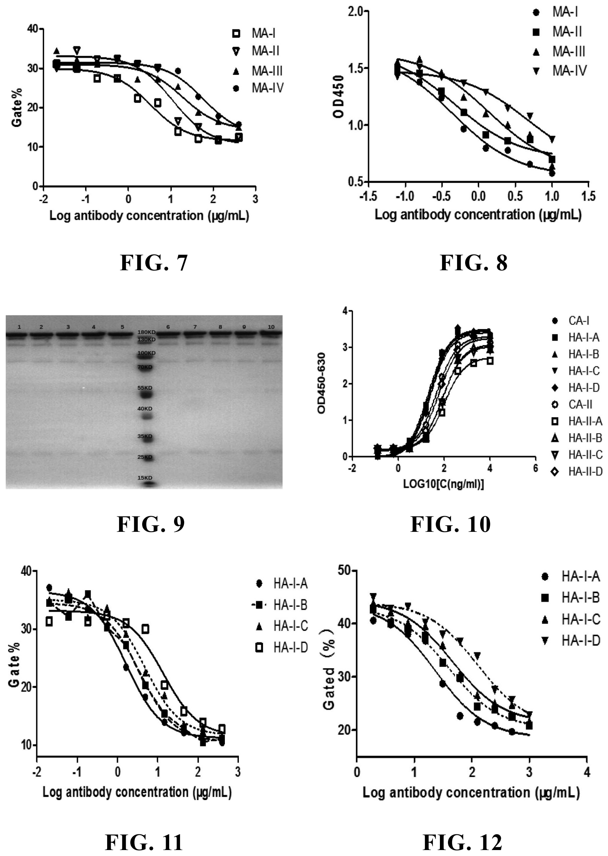

Based on the above data and FIG. 7, it can be seen that the four different screened murine candidate molecules can all effectively inhibit the binding of the IL-11 ligand protein to the receptor IL-11RA on the cell surface.

### Example 9 Inhibition of TIMP-1 secretion in embryonic lung fibroblast MRC-5 cells by murine antibodies

After trypsin digestion, the embryonic lung fibroblast MRC-5 cells were counted. A certain number of cells were taken, centrifuged, and resuspended in MEM complete medium (purchased from GIBCO, Catalog No.: 10370-021). The cell density was adjusted to 2E + 5 cells/mL, and the cells were added to a 96-well plate at 100 µL/well. IL-11-mFc ligand protein was diluted with MEM complete medium to a concentration of 16 µg/mL, and added to the corresponding 96-well plate at 50 µL/well. The four murine antibody molecules MA-I, MA-II, MA-III, and MA-IV were diluted serially with MEM complete medium, wherein the initial concentration was 40 µg/mL, and the antibody molecules were subject to 2-fold serial dilution for a total of 8 gradients. They were added at 50 µL/well to the 96-well plate containing the mixture of the cell suspension and IL-11-mFc ligand protein. After gentle mixing, the plate was placed in a 37 °C CO₂ incubator and incubated overnight for about 20 hours. The cell culture supernatant was taken and detected using a TIMP-1 ELISA kit (purchased from ExCell Biotechnology Co., Ltd., Catalog No.: EH021-96).

Human TIMP-1 detection kit: Cell supernatant and standards were added to the sample wells at 100 µL/well. Biotinylated antibody working solution (1:100 dilution) was immediately added at 50 µL/well. The plate was sealed with a sealing film and incubated with shaking at room temperature for 2 hours. After incubation, the plate was washed 4 times with wash buffer. Enzyme conjugate working solution (1:100 dilution) from the TIMP-1 detection kit was added at 100 µL/well. The plate was sealed with a sealing film and incubated with shaking at room temperature for 1 hour. After incubation, the plate was washed 4 times with wash buffer. TMB color development solution was added at 100 µL/well, and the plate was incubated protected from light at room temperature for about 15 minutes. The reaction was stopped with stop solution at 100 µL/well. The plate was read at 450 nm using a microplate reader, and the corresponding IC₅₀ was calculated. The specific data are as follows:

| **Clone** | MA-I | MA-II | MA-III | MA-IV |
|---|---|---|---|---|
| IC50 (µg/mL) | 0.453 | 0.475 | 1.356 | 4.601 |

Based on the above data and FIG. 8, it can be seen that the four different screened murine candidate molecules can all effectively inhibit the release of TIMP-1 from human embryonic lung fibroblast MRC-5 cells stimulated by the IL-11 ligand protein.

### Example 10

Example 10 of the present application further defines that the antibody or the antigen-binding fragment thereof is a chimeric antibody molecule. The chimeric antibody molecule further comprises a human antibody constant region. The human antibody constant region comprises a human antibody heavy chain constant region and a human antibody light chain constant region. The amino acid sequence of the human antibody heavy chain constant region is as set forth in one of SEQ ID NO: 27, SEQ ID NO: 28, or SEQ ID NO: 29. The amino acid sequence of the human antibody light chain constant region is as set forth in SEQ ID NO: 30.
SEQ ID NO: 27 (the amino acid sequence of the heavy chain constant region of human IgG1 type): 
ASTKGPSVFPLAPSSKSTSGGTAALGCLVKDYFPEPVTVSWNSGALTSGVHTFPAVLQS
SGLYSLSSVVTVPSSSLGTQTYICNVNHKPSNTKVDKRVEPKSCDKTHTCPPCPAPEAAGGP
SVFLFPPKPKDTLMISRTPEVTCVVVDVSHEDPEVKFNWYVDGVEVHNAKTKPREEQYNST
YRVVSVLTVLHQDWLNGKEYKCKVSNKALPAPIEKTISKAKGQPREPQVYTLPPSREEMTK
NQVSLTCLVKGFYPSDIAVEWESNGQPENNYKTTPPVLDSDGSFFLYSKLTVDKSRWQQGN
VFSCSVMHEALHNHYTQKSLSLSPGK;
SEQ ID NO: 28 (the amino acid sequence of the heavy chain constant region of human IgG2 type): 
ASTKGPSVFPLAPCSRSTSESTAALGCLVKDYFPEPVTVSWNSGALTSGVHTFPAVLQSS
GLYSLSSVVTVPSSNFGTQTYTCNVDHKPSNTKVDKTVERKCCVECPPCPAPPVAGPSVFLF
PPKPKDTLMISRTPEVTCVVVDVSHEDPEVQFNWYVDGVEVHNAKTKPREEQFNSTFRVVS
VLTVVHQDWLNGKEYKCKVSNKGLPAPIEKTISKTKGQPREPQVYTLPPSREEMTKNQVSL
TCLVKGFYPSDIAVEWESNGQPENNYKTTPPMLDSDGSFFLYSKLTVDKSRWQQGNVFSCS
VMHEALHNHYTQKSLSLSPGK;
SEQ ID NO: 29 (the amino acid sequence of the heavy chain constant region of human IgG4 type): 
ASTKGPSVFPLAPCSRSTSESTAALGCLVKDYFPEPVTVSWNSGALTSGVHTFPAVLQSS
GLYSLSSVVTVPSSSLGTKTYTCNVDHKPSNTKVDKRVESKYGPPCPPCPAPEFLGGPSVFLF
PPKPKDTLMISRTPEVTCVVVDVSQEDPEVQFNWYVDGVEVHNAKTKPREEQFNSTYRVVS
VLTVLHQDWLNGKEYKCKVSNKGLPSSIEKTISKAKGQPREPQVYTLPPSQEEMTKNQVSL
TCLVKGFYPSDIAVEWESNGQPENNYKTTPPVLDSDGSFFLYSRLTVDKSRWQEGNVFSCSV
MHEALHNHYTQKSLSLSLGK;
SEQ ID NO: 30 (the amino acid sequence of the light chain constant region of human C_{κ} chain):


.

### Example 11 Preparation of chimeric antibody molecules

Example 11 of the present application further defines, on the basis of Example 10, that the human antibody constant region comprises the heavy chain constant region of human IgG1 (its amino acid sequence is as set forth in SEQ ID NO: 27) and the light chain constant region of human C_{κ} (its amino acid sequence is as set forth in SEQ ID NO: 30).

Specific preparation method:
The heavy chain variable region VH (SEQ ID NO: 16) of the murine antibody molecules MA-I and MA-II, the light chain variable region VL gene of MA-I (SEQ ID NO: 17), and the light chain variable region VL gene of MA-II (SEQ ID NO: 18), obtained by screening the immune phage antibody library in Example 2, were kept unchanged as murine sequences. They were separately cloned into the vector pTSE containing heavy chain constant region and light chain constant region genes (as shown in FIG. 3). The heavy chain constant region is human IgG1 type (the amino acid sequence is as set forth in SEQ ID NO: 27), and the light chain constant region is human C_{κ} type (the amino acid sequence is as set forth in SEQ ID NO: 30). HEK293E cells (purchased from: Institute of Basic Medical Sciences of Chinese Academy of Medical Sciences, Catalog No.: GNHu43) were transiently transfected for antibody expression to obtain the chimeric antibodies CA-I and CA-II.

### Example 12 Humanization of murine antibody molecules

First, the sequences of the murine antibody molecules MA-I and MA-II from Example 2 were compared with the human antibody germline database (v-base) to identify human antibody light and heavy chain germlines with high homology as candidate sequences. Then, the CDR sequences of the murine antibody molecules MA-I and MA-II were grafted onto the human candidate sequences for homology modeling. Subsequently, key framework amino acid residues that may be important for maintaining the CDR loop structure were identified through three-dimensional structure simulation, and back-mutations for the humanized antibodies were designed accordingly. The designed light and heavy chain variable region sequences of the humanized antibodies containing back-mutations were optimized and synthesized by Nanjing Genscript Biotechnology Co., Ltd., China, and then ligated into transient expression vectors. The combinations of light and heavy chains obtained from humanization were analyzed. For MA-I, the following humanized antibody molecules were obtained: HA-I-A, HA-I-B, HA-I-C, and HA-I-D. For MA-II, the following humanized antibody molecules were obtained: HA-II-A, HA-II-B, HA-II-C, and HA-II-D. The sequences of the eight monoclonal antibodies obtained from the above screening are as follows:

| **Monoclonal Antibody** | **Heavy Chain Variable Region Sequence** | **Light Chain Variable Region Sequence** |
|---|---|---|
| HA-I-A | SEQ ID No:31 | SEQ ID No:32 |
| HA-I-B | SEQ ID No:33 | SEQ ID No: 34 |
| HA-I-C | SEQ ID No: 31 | SEQ ID No: 35 |
| HA-I-D | SEQ ID No: 36 | SEQ ID No: 37 |
| HA-II-A | SEQ ID No:31 | SEQ ID No: 38 |
| HA-II-B | SEQ ID No:31 | SEQ ID No: 39 |
| HA-II-C | SEQ ID No:33 | SEQ ID No: 39 |
| HA-II-D | SEQ ID No:36 | SEQ ID No:40 |

Specifically, SEQ ID NO: 31 (the amino acid sequence of the heavy chain variable region of HA-I-A, HA-I-C, HA-II-A, and HA-II-B): 
QVQLVESGGGLVKPGGSLRLSCAASGFTFSDYYMFWVRQAPGKGLEWVATISDGGTY
TYYPDSVKGRFTISRDNAKNSLYLQMNSLRAEDTAVYYCARDGGYVSSPEAMDYWGQGTL
VTVSS;
SEQ ID NO: 32 (the amino acid sequence of the light chain variable region of HA-I-A): 
DIVLTQSPSSLSASVGDRVTITCRASQDISNYLNWYQQKPGKAPKLLIYYTSRLIISGVPS
RFSGSGSGTDFTFTISSLQPEDIATYYCQQGNTLPPTFGGGTKVEIK;
SEQ ID NO: 33 (the amino acid sequence of the heavy chain variable region of HA-I-B and HA-II-C): 
QVQLVESGGGLVKPGGSLRLSCAASGFTFSDYYMFWVRQAPGKGLEWVSTISDGGTY
TYYPDSVKGRFTISRDNAKNSLYLQMNSLRAEDTAVYYCARDGGYVSSPEAMDYWGQGTL
VTVSS;
SEQ ID NO: 34 (the amino acid sequence of the light chain variable region of HA-I-B): 
DIVLTQSPSSLSASVGDRVTITCRASQDISNYLNWYQQKPGKAPKLLIYYTSRLHSGVPS
RFSGSGSGTDYTFTISSLQPEDIATYYCQQGNTLPPTFGGGTKVEIK;
SEQ ID NO: 35 (the amino acid sequence of the light chain variable region of HA-I-C): 
DIVLTQSPSSLSASVGDRVTITCRASQDISNYLNWYQQKPGKAVKLLIYYTSRLHSGVPS
RFSGSGSGTDYTFTISSLQPEDIATYFCQQGNTLPPTFGGGTKVEIK;
SEQ ID NO: 36 (the amino acid sequence of the heavy chain variable region of HA-I-D and HA-II-D): 
QVQLVESGGGLVKPGGSLRLSCAASGFTFSDYYMFWVRQAPGKGLEWVATISDGGTY
TYYPDSVKGRFTISRDNAKNSLYLQMNSLRAEDTAMYYCARDGGYVSSPEAMDYWGQGT
SVTVSS;
SEQ ID NO: 37 (the amino acid sequence of the light chain variable region of HA-I-D): 
DIVLTQSPSSLSASVGDRVTITCRASQDISNYLNWYQQKPGGAVKLLIYYTSRLHSGVPS
RFSGSGSGTDYTFTISSLQPEDIATYFCQQGNTLPPTFGGGTKVEIK;
SEQ ID NO: 38 (the amino acid sequence of the light chain variable region of HA-II-A): 
DIVLTQSPSSLSASVGDRVTITCRASQDISNYLNWYQQKPGKAPKLLIYYTSRLIISGVPS
RFSGSGSGTDFTFTISSLQPEDIATYYCQQGNTLPWTFGGGTKVEIK;
SEQ ID NO: 39 (the amino acid sequence of the light chain variable region of HA-II-B and HA-II-C): 
DIVLTQSPSSLSASVGDRVTITCRASQDISNYLNWYQQKPGKAPKLLIYYTSRLHSGVPS
RFSGSGSGTDYTFTISSLQPEDIATYYCQQGNTLPWTFGGGTKVEIK;
SEQ ID NO: 40 (the amino acid sequence of the light chain variable region of HA-II-D):

DIVLTQSPSSLSASVGDRVTITCRASQDISNYLNWYQQKPGGTVKLLIYYTSRLHSGVP
SRFSGSGSGTDYTFTISSLQPEDIATYFCQQGNTLPWTFGGGTKVEIK.

### Example 13

Example 13 of the present application further defines, on the basis of Example 12, that the human antibody constant region comprises a human antibody heavy chain constant region and a human antibody light chain constant region. The amino acid sequence of the human antibody heavy chain constant region is as set forth in one of SEQ ID NO: 27, SEQ ID NO: 28, or SEQ ID NO: 29. The amino acid sequence of the human antibody light chain constant region is as set forth in SEQ ID NO: 30.

The specific sequences of the above human antibody constant regions are the same as those in Example 10.

### Example 14 Preparation of humanized antibody molecules

Example 14 of the present application further defines, on the basis of Example 13, that the human antibody constant region comprises the heavy chain constant region of human IgG1 type (its amino acid sequence is as set forth in SEQ ID NO: 27) and the light chain constant region of human C_{κ} type(its amino acid sequence is as set forth in SEQ ID NO: 30).

The encoding genes for the heavy chain VH and light chain VL of the eight humanized antibody molecules HA-I-A, HA-I-B, HA-I-C, HA-I-D, HA-II-A, HA-II-B, HA-II-C, and HA-II-D obtained from humanization in Example 12 were separately cloned into the vector pTSE containing heavy chain constant region and light chain constant region genes (as shown in FIG. 3). The heavy chain constant region is human IgG1 type (the amino acid sequence is as set forth in SEQ ID NO: 27), and the light chain constant region is the C_{κ} chain (the amino acid sequence is as set forth in SEQ ID NO: 30).

The two chimeric antibodies CA-I and CA-II obtained in Example 11 and the eight humanized antibody molecules HA-I-A, HA-I-B, HA-I-C, HA-I-D, HA-II-A, HA-II-B, HA-II-C, and HA-II-D obtained in Example 12 were transiently transfected into HEK293 cells (purchased from the Institute of Basic Medical Sciences of Chinese Academy of Medical Sciences, Catalog No.: GNHu43) for antibody expression. Monoclonal antibodies were purified using an AKTA instrument with a protein A affinity column. Protein concentration was determined using a BCA kit (purchased from: Beijing Huitian Dongfang Science and Technology Co., Ltd., Beijing, China, Catalog No.: BCA0020). Subsequently, protein size was identified by SDS-PAGE, and the results are shown in FIG. 9. From left to right: non-reduced protein molecular weights of HA-I-A, HA-I-B, HA-I-C, HA-I-D, and the chimeric antibody CA-I prepared in Example 11, and reduced protein molecular weights of marker, HA-II-A, HA-II-B, HA-II-C, HA-II-D, and the chimeric antibody CA-II. The molecular weight of each band was consistent with the theoretical value.

### Example 15

Example 15 defines that the antibody or the antigen-binding fragment thereof disclosed in the present application is one of Fab, F(ab')2, Fv or ScFv, or a combination thereof, and is not limited to the monoclonal antibodies disclosed in the present application.

### Example 16

Example 16 of the present application also provides a nucleic acid molecule encoding the antibody binding to IL-11 or the antigen-binding fragment thereof.

The present application also provides a recombinant DNA expression vector comprising the above nucleic acid molecule.

The present application also provides a host cell transfected with the above recombinant DNA expression vector. The host cell is a prokaryotic cell, a yeast cell, an insect cell, or a mammalian cell.

Further preferably, the host cell is a mammalian cell, and the mammalian cell is a HEK293 cell, a CHO cell, or an NS0 cell.

The present application also provides a drug comprising the antibody binding to IL-11 or the antigen-binding fragment thereof.

The present application also provides a detection reagent for detecting the antibody binding to IL-11 or the antigen-binding fragment thereof.

The present application also provides a combined drug composition comprising the antibody binding to IL-11 or the antigen-binding fragment thereof, and further comprising a second composition selected from the group consisting of an antibody binding to PD-1, PD-L1, VEGF, VEGFR, TNF-α, or TSLP and an antigen-binding fragment thereof.

Preferably, the antibody binding to PD-1 or the antigen-binding fragment thereof is selected from the group consisting of DFPD1-9, DFPD1-10, DFPD1-11, DFPD1-12, DFPD1-13, Nivolumab, Pembrolizumab, Toripalimab, Sintilimab, Tislelizumab, Camrelizumab, Penpulimab, and Zimberelimab, wherein the heavy chain constant region and light chain constant region sequences of DFPD1-9, DFPD1-10, DFPD1-11, DFPD1-12, and DFPD1-13 are the same as those of the anti-PD-1 monoclonal antibody provided in the patent application No. CN201510312910.8.

Preferably, the antibody binding to VEGF or the antigen-binding fragment thereof is selected from the group consisting of Aflibercept, Bevacizumab, and Ranibizumab.

### Example 17

Example 17 of the present application provides use of the antibody binding to IL-11 or the antigen-binding fragment thereof in the manufacture of a medicament for treating or preventing a fibrotic disease, inflammation, cancer, or an autoimmune disease in humans.

Preferably, the fibrotic disease includes but is not limited to fibrosis of heart, liver, kidney, lung, gallbladder, bladder, stomach, bone marrow, penis, breast, blood vessel, eye, pancreas, spleen, brain, intestine, muscle, or skin.

Preferably, the inflammation includes but is not limited to hepatitis, myocarditis, nephritis, pneumonia, cholecystitis, cystitis, gastritis, osteomyelitis, prostatitis, mastitis, pancreatitis, enteritis, arthritis, polymyositis, dermatomyositis, or dermatitis.

Preferably, the cancer includes but is not limited to leukemia, lung cancer, gastric cancer, esophageal cancer, ovarian cancer, head and neck cancer, melanoma, renal cancer, breast cancer, colorectal cancer, liver cancer, pancreatic cancer, or bladder cancer.

Preferably, the autoimmune disease includes but is not limited to psoriasis, Crohn's disease, primary biliary cirrhosis, systemic lupus erythematosus, or multiple sclerosis.

The present application also provides a method for treating or preventing a fibrotic disease, inflammation, cancer, or an autoimmune disease in a human subject, comprising administering to the subject a therapeutically or prophylactically effective amount of the antibody binding to IL-11 or the antigen-binding fragment thereof.

### Example 18 Binding experiment of humanized antibody molecules with IL-11

IL-11 antigen was coated overnight at 4 °C using a pH 9.6 carbonate buffer at 100 ng/well/100 µL. After washing five times with 300 µL/well of PBST, 1% BSA-PBST was added at 280 µL/well, and the plate was blocked at 37 °C for 1 hour. The humanized antibodies HA-I-A, HA-I-B, HA-I-C, HA-I-D, HA-II-A, HA-II-B, HA-II-C, HA-II-D and the chimeric antibodies CA-I, CA-II prepared in Example 11 were diluted with 1% BSA-PBST, wherein the initial concentration of the humanized antibodies was 10 µg/mL, and the antibodies were subjected to 5-fold serial dilution for a total of 8 gradients. The plate was incubated at 37 °C for 1 hour. After washing five times with 300 µL/well of PBST, goat anti-human IgG Fab HRP (purchased from Invitrogen, Catalog No.: 31482) diluted 1:5000 with 1% BSA-PBST was added, and the plate was incubated at 37 °C for 1 hour. Color development was performed for 5 minutes at room temperature using a TMB color development kit at 100 µL/well, and then stopped with 2 M H₂SO₄. The plate was read at 450 nm/630 nm using a microplate reader, and the corresponding EC50 was calculated. The specific data are as follows:

| **Clone** | EC50 (ng/ml) |
|---|---|
| CA-I | 22.55 |
| HA-I-A | 20.44 |
| HA-I-B | 23.24 |
| HA-I-C | 25.21 |
| HA-I-D | 26.15 |
| CA-II | 46.35 |
| HA-II-A | 101.8 |
| HA-H-B | 88.29 |
| HA-II-C | 84.89 |
| HA-II-D | 58.51 |

Based on the above data and experimental results as shown in FIG. 10, all eight different humanized antibody molecules can bind to IL-11. The EC50 of the humanized antibody molecules HA-I-A, HA-I-B, HA-I-C, and HA-I-D are all close to that of the chimeric antibody CA-I, and the EC50 of the humanized antibody molecules HA-II-A, HA-II-B, HA-II-C, and HA-II-D are all close to that of the chimeric antibody CA-II. These indicate that the humanized antibody molecules retain the high binding ability to IL-11 of the murine parent antibodies MA-I and MA-II.

### Example 19 Inhibition of IL-11 binding to the IL-11RA receptor on the surface of BaF/3-IL-11RA cells by humanized antibody molecules

Four humanized antibody molecules with superior protein-level binding activity, HA-I-A, HA-I-B, HA-I-C, and HA-I-D, were selected for cell-based activity evaluation experiments. BaF/3-IL-11RA cell strain was counted. A certain number of cells were taken, centrifuged, and resuspended in PBS buffer. The cell density was adjusted to 1E + 6 cells/mL, and the cells were added to a 96-well plate at 100 µL/well. IL-11-mFc ligand protein was diluted with PBS to a concentration of 18 µg/mL, and added at 50 µL/well to the corresponding positions of the 96-well plate containing BaF/3-IL-11RA cells. After gentle mixing, the 96-well plate was placed at 4 °C and incubated for 1 hour. The four humanized antibody molecules HA-I-A, HA-I-B, HA-I-C, and HA-I-D were diluted serially with PBS buffer, wherein the initial concentration was 800 µg/mL, and the antibody molecules were subjected to 3-fold serial dilution for a total of 10 gradients. They were added at 50 µL/well to the corresponding positions of the 96-well plate containing the mixture of BaF/3-IL-11RA cells and IL-11-mFc ligand protein. After mixing evenly, the plate was incubated at 4 °C for 2 hours. After incubation, centrifugation was performed at 3000 rpm, the cells were washed once with PBS buffer, and the cell pellet was collected. Pre-prepared goat anti-mouse IgG human ads-FITC antibody (purchased from SouthernBiotech, Catalog No.: 1030-02) was added to the cell pellet, and the plate was incubated at 4 °C for 30 minutes. After centrifugation at 3000 rpm, the cells were washed once, resuspended in 100 µL PBS, and analyzed by flow cytometry. Fluorescence signals in the FL1-A channel were collected. The dose-response curve was plotted, and the corresponding IC50 was calculated. The specific data are as follows:

| **Clone** | IIA-I-A | IIA-I-B | IIA-I-C | IIA-I-D |
|---|---|---|---|---|
| IC50 (µg/mL) | 1.675 | 3.578 | 4.869 | 13.76 |

Based on the above data and FIG. 11, it can be seen that the four screened humanized candidate molecules can all inhibit the binding of the IL-11 ligand protein to the receptor IL-11RA on the surface of BaF/3-IL-11RA cells.

### Example 20 Inhibition of IL-11 binding to the GP130 receptor on the surface of BaF/3-GP130 cells by humanized antibody molecules

BaF/3-GP130 cell strain was counted. A certain number of cells were taken, centrifuged, and resuspended in PBS buffer. The cell density was adjusted to 1E + 6 cells/mL, and the cells were added to a 96-well plate at 100 µL/well. IL-11-mFc ligand protein was diluted with PBS to a concentration of 12 µg/mL, and added at 50 µL/well to the corresponding positions of the 96-well plate containing BaF/3-GP130 cells. After gentle mixing, the 96-well plate was placed at 4 °C and incubated for 1 hour. The four humanized antibody molecules HA-I-A, HA-I-B, HA-I-C, and HA-I-D were diluted serially with PBS buffer, wherein the initial concentration was 2000 µg/mL, and the antibody molecules were subjected to 2-fold serial dilution for a total of 10 gradients. They were added at 50 µL/well to the corresponding positions of the 96-well plate containing BaF/3-GP130 cells and IL-11-mFc ligand protein. After mixing evenly, the 96-well plate was placed at 4 °C and incubated for 2 hours. After incubation, centrifugation was performed at 3000 rpm, the cells were washed once with PBS buffer, and the cell pellet was collected. Pre-prepared goat anti-mouse IgG human ads-FITC antibody (purchased from SouthernBiotech, Catalog No.: 1030-02) was added to the cell pellet at 100 µL/well, and the plate was incubated at 4 °C for 30 minutes. After centrifugation at 3000 rpm, the cells were washed once with PBS buffer, resuspended in PBS buffer at 100 µL/well, and analyzed by flow cytometry. Fluorescence signals in the FL1-A channel were collected. The dose-response curve was plotted, and the corresponding IC₅₀ was calculated. The specific data are as follows:

| **Clone** | HA-I-A | HA-I-B | HA-I-C | HA-I-D |
|---|---|---|---|---|
| IC50 (µg/mL) | 23.49 | 39.57 | 46.19 | 127.6 |

Based on the above data and FIG. 12, it can be seen that the four screened humanized candidate molecules can all block the binding of the IL-11 ligand protein to the receptor GP130 on the surface of BaF/3-GP130 cells.

### Example 21 Biological activity detection of humanized antibody molecules (reporter gene)

The BaF/3-GP130-STAT3-Luc engineered cell strain (construction method of the BaF/3-GP130-STAT3-Luc engineered cell strain: the plasmid GP130 (purchased from Sino Biological Inc., Catalog No.: HG10974-UT) and the plasmid STAT3-Luc-NeoR (purchased from Genomeditech (Shanghai) Co., Ltd., Catalog No.: GM-021003) were separately electroporated into BAF/3 cells (purchased from the National Laboratory Cell Resources Center) in two separate steps, and screening was performed to obtain the BaF/3-GP130-STAT3-Luc engineered cell strain) was counted. The cell density was adjusted to 2E + 6 cells/mL using sample dilution buffer (composed of 90% IMDM, 10% FBS, and 10 ng/mL mouse IL-3). After gentle mixing, the cell suspension was added to a 96-well plate at 50 µL/well. The four humanized antibody molecules HA-I-A, HA-I-B, HA-I-C, and HA-I-D were separately diluted with sample dilution buffer to an initial concentration of 200 µg/ml, and subjected to 5-fold serial dilution for a total of 10 gradients. They were added at 100 µL/well to the corresponding positions of the 96-well plate containing the engineered cell strain. Two replicate wells were set for each sample concentration. IL-11 protein was prepared at a concentration of 10 µg/mL with sample dilution buffer, and added at 50 µL/well to the 96-well plate containing the engineered cell strain and humanized antibody molecules. The cell culture plate was gently mixed and placed in a 37 °C CO₂ incubator for 6 hours. After removing the supernatant by centrifugation, lysis buffer was added to a 384-well plate at 10 µL/well, and an equal amount of luciferase reaction substrate (purchased from Promega Biotechology Co., Ltd., Catalog No.: E2610) was added. The reaction was performed at room temperature for 5 minutes. The fluorescence value was read using a microplate reader, and the corresponding IC₅₀ was calculated. The specific data are as follows:

| **Candidate Molecule** | HA-I-A | HA-I-B | HA-I-C | HA-I-D |
|---|---|---|---|---|
| IC50 (µg/mL) | 0.045 | 0.103 | 0.152 | 0.465 |

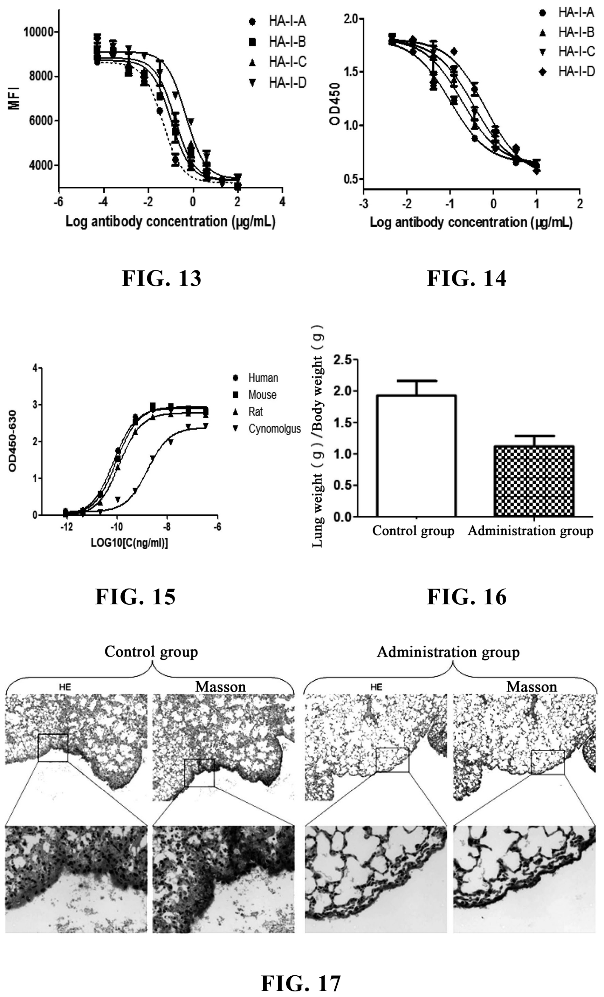

Based on the above data and as shown in FIG. 13, the four screened humanized antibody molecules can all block the binding of IL-11 to the receptors IL-11RA and GP130, thereby inhibiting the transmission of signaling pathways.

### Example 22 Inhibition of TIMP-1 secretion in embryonic lung fibroblast MRC-5 cells by humanized antibody molecules

After trypsin digestion, embryonic lung fibroblast MRC-5 cells were counted. A certain number of cells were taken, centrifuged, and resuspended in MEM complete medium. The cell density was adjusted to 2E + 5 cells/mL, and the cells were added to a 96-well plate at 100 µL/well. IL-11-mFc ligand protein was diluted with MEM complete medium to a concentration of 16 µg/mL, and added to the corresponding 96-well plate at 50 µL/well. The four humanized antibody molecules HA-I-A, HA-I-B, HA-I-C, and HA-I-D were diluted serially with MEM complete medium, wherein the initial concentration was 40 µg/mL, and the antibody molecules were subjected to 3-fold serial dilution for a total of 8 gradients. They were added at 50 µL/well to the 96-well plate containing the mixture of the cell suspension and IL-11-mFc ligand protein. After gentle mixing, the plate was placed in a 37 °C CO₂ incubator and incubated overnight for about 20 hours. The cell culture supernatant was taken and detected using a TIMP-1 ELISA kit (method same as Example 9). The plate was read at 450 nm using a microplate reader, and the corresponding IC₅₀ was calculated. The specific data are as follows:

| **Clone** | HA-I-A | HA-I-B | HA-I-C | HA-I-D |
|---|---|---|---|---|
| IC50 (µg/mL) | 0.101 | 0.190 | 0.343 | 0.657 |

Based on the above data and FIG. 14, it can be seen that the IL-11 ligand protein stimulates the release of TIMP-1 from human embryonic lung fibroblast MRC-5 cells, which can be effectively inhibited by the four screened humanized antibody molecules.

### Example 23 Cross-binding assay of humanized antibody molecules with IL-11 from different species

The humanized antibody molecule with superior activity in protein-level and functional assays, HA-I-A, was selected for cross-binding assay with IL-11 from different species. Human IL-11, mouse IL-11 (purchased from Sino Biological Inc., Beijing, China, Catalog No.: 50117-MNCE), rat IL-11 (purchased from Kanglang Biotechnology, Catalog No.: KL40001Ra), and cynomolgus monkey IL-11 (purchased from Sino Biological Inc., Catalog No.: 90925-CNCE) were separately coated overnight at 4 °C using a pH 9.6 carbonate buffer at 100 ng/well/100 µL. After washing five times with 300 µL/well of PBST, 1% BSA-PBST was added at 280 µL/well, and the plate was blocked at 37 °C for 1 hour. The humanized antibody HA-I-A was diluted with 1% BSA-PBST, wherein the initial concentration was 50 µg/mL, and the antibody was subjected to a 5-fold serial dilution for a total of 9 gradients, with two replicate wells per gradient. It was added to the 96-well plate at 100 µL/well, and the plate was incubated at 37 °C for 1 hour. After washing five times with PBST at 300 µL/well, goat anti-human IgG Fab HRP (purchased from Invitrogen, Catalog No.: 31482) was diluted with 1% BSA-PBST to a working concentration of 1:5000, added to the 96-well plate at 100 µL/well, and the plate was incubated at 37 °C for 1 hour. After washing five times with PBST at 300 µL/well, color development was performed protected from light at room temperature for 5 minutes using a TMB color development kit at 100 µL/well, and then stopped with 2 M H₂SO₄. The plate was read at 450 nm/630 nm using a microplate reader, and the corresponding EC₅₀ was calculated. The specific data are as follows:

| **Clone** HA-I-A | IL-11 (Human) | IL-11 (Mouse) | IL-11 (Rat) | IL-11 (Cynomolgus) |
|---|---|---|---|---|
| EC50 (ng/ml) | 10.65 | 13.89 | 17.88 | 238.7 |

Based on the above data and as shown in FIG. 15, the humanized antibody molecule HA-I-A can all bind to human IL-11, mouse IL-11, rat IL-11, and cynomolgus monkey IL-11 with high affinity.

### Example 24 Therapeutic efficacy experiment of anti-IL-11 monoclonal antibody on pulmonary fibrosis

Bleomycin (bLF) was used to model and study the therapeutic effect of the anti-IL-11 monoclonal antibody HA-I-A on pulmonary fibrosis.
Animal species: C57BL/6J mice (purchased from GemPharmatech Biotechnology Co., Ltd., Jiangsu, China);
number, gender, and age: 6 mice/group, male, 6 to 8 weeks old;
the control group was injected with physiological saline only;
the administration group was injected with the HA-I-A antibody molecule twice a week for 4 weeks.

Body weight was measured weekly, and animals were observed for abnormalities; organ weight detection: lungs were collected, lung weight was measured, and the lung-to-body weight ratio was calculated; pulmonary pathology detection: lungs were sectioned, and the degree of pulmonary fibrosis was observed by hematoxylin and eosin (HE) staining and Masson's staining.

As shown in FIG. 16, after administration of the HA-I-A antibody molecule, the lung-to-body weight ratio in the administration group was significantly lower than that in the control group; as shown in FIG. 17, compared to the control group, lung sections of the administration group showed significantly reduced pulmonary fibrosis, indicating that the anti-IL-11 monoclonal antibody HA-I-A molecule can effectively inhibit the development of pulmonary fibrosis.

### Example 25 Therapeutic efficacy experiment of anti-IL-11 monoclonal antibody on cardiac fibrosis

Isoproterenol was used to model and study the therapeutic effect of the anti-IL-11 monoclonal antibody HA-I-A on cardiac fibrosis.
Animal species: C57BL/6J mice (purchased from GemPharmatech Biotechnology Co., Ltd., Jiangsu, China);
number, gender, and age: 6 mice/group, male, 6 to 8 weeks old;
the control group was injected with physiological saline only;
the administration group was injected with the HA-I-A antibody molecule twice a week for 4 weeks.

Body weight was measured weekly, and animals were observed for abnormalities; organ weight detection: hearts were collected, heart weight was measured, and the heart-to-body weight ratio was calculated; cardiac pathology detection: hearts were sectioned, and the degree of cardiac fibrosis was observed by hematoxylin and eosin (HE) staining and Masson's staining.

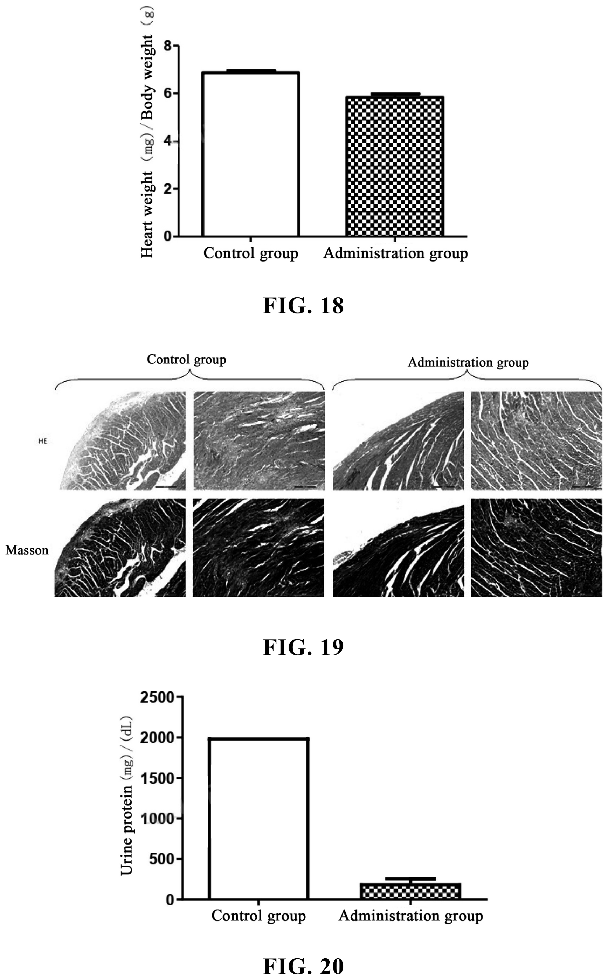

As shown in FIG. 18, the heart-to-body weight ratio in the administration group was significantly lower than that in the control group; as shown in FIG. 19, compared to the control group, heart sections of the administration group showed significantly reduced cardiac fibrosis, indicating that the anti-IL-11 monoclonal antibody HA-I-A molecule can effectively inhibit the development of cardiac fibrosis.

### Example 26 Therapeutic efficacy experiment of anti-IL-11 monoclonal antibody on renal fibrosis

Doxorubicin (dKF) was used to model and study the therapeutic effect of the anti-IL-11 monoclonal antibody HA-I-A on renal fibrosis.
Animal species: BALB/c mice (purchased from GemPharmatech Biotechnology Co., Ltd., Jiangsu, China);
number, gender, and age: 6 mice/group, male, 6 to 8 weeks old;
the control group was injected with physiological saline only;
the administration group was injected with the HA-I-A antibody molecule twice a week for 4 weeks.

Body weight was measured weekly, and animals were observed for abnormalities; organ weight detection: kidneys were collected, kidney weight was measured, and urine protein content was detected; renal pathology detection: kidneys were sectioned, and the degree of renal fibrosis was observed by hematoxylin and eosin (HE) staining and Masson's staining.

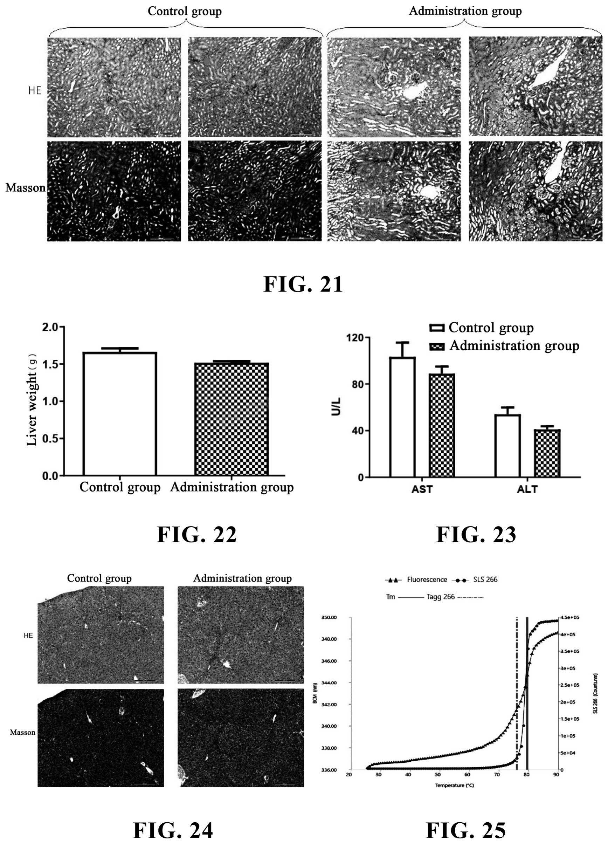

As shown in FIG. 20, compared to the control group, the urine protein content in the kidneys of the administration group was significantly lower; as shown in FIG. 21, compared to the control group, kidney sections of the administration group showed significantly reduced renal fibrosis, indicating that the anti-IL-11 monoclonal antibody HA-I-A molecule can effectively inhibit the development of renal fibrosis.

### Example 27 Therapeutic efficacy experiment of anti-IL-11 monoclonal antibody on hepatic fibrosis

CCl₄ was used to model and study the therapeutic effect of the anti-IL-11 monoclonal antibody HA-I-A on hepatic fibrosis.
Animal species: C57BL/6J mice (purchased from GemPharmatech Biotechnology Co., Ltd., Jiangsu, China);
number, gender, and age: 6 mice/group, male, 6 to 8 weeks old;
the control group was injected with physiological saline only;
the administration group was injected with the HA-I-A antibody molecule twice a week for 4 weeks.

Body weight monitoring: body weight was measured weekly, and animals were observed for abnormalities; hepatic pathology detection: livers were sectioned, and the degree of hepatic fibrosis was observed by hematoxylin and eosin (HE) staining and Masson's staining; organ weight detection: livers were collected, liver weight was measured, and HE staining was performed; serum detection: serum was collected, and the levels of alanine aminotransferase (ALT) and aspartate aminotransferase (AST) in mouse serum were detected.

As shown in FIG. 22, compared to the control group, the liver weight in the administration group was significantly lower; as shown in FIG. 23, the levels of ALT and AST in the serum of the administration group were significantly lower than those in the control group; as shown in FIG. 24, compared to the control group, liver sections of the administration group showed significantly reduced hepatic fibrosis, indicating that the anti-IL-11 monoclonal antibody HA-I-A molecule can effectively inhibit the development of hepatic fibrosis.

### Example 28 Thermal stability assessment of anti-IL-11 monoclonal antibody HA-I-A

The thermal stability of the anti-IL-11 monoclonal antibody HA-I-A was evaluated using a multi-functional protein thermal stability analysis system (purchased from Unchained Labs). Conformational changes of the protein were detected by monitoring intrinsic fluorescence of the protein as a function of temperature (from 25 °C to 95 °C at a heating rate of 0.3 °C/min) to determine the protein melting temperature Tm and assess protein conformational stability. When protein aggregation occurs, it causes interference in scattered light waves, leading to an increase in scattered light signal. Colloidal stability of the protein was determined by static light scattering (characterized by Tagg). The results are shown in the following table and FIG. 25.

| **Sample** | Tm (°C) | Tagg 266 (°C) |
|---|---|---|
| **12.5 mg/ml of Anti-IL-11 Monoclonal Antibody HA-I-A** | 79.5 | 75.9 |

The melting temperature Tm of the anti-IL-11 monoclonal antibody HA-I-A was 79.5 °C, and the average Tagg was 75.9 °C, demonstrating favorable conformational stability and colloidal stability.

The present application is not limited to the above optimal embodiments. Any individual, inspired by the present application, may derive various other forms of products. However, regardless of any modifications made to their shape or structure, any technical solution that is identical or similar to that of the present application shall fall within the scope of protection of the present application.

## Claims

1. An antibody binding to IL-11 or an antigen-binding fragment thereof, comprising three heavy chain complementarity determining regions represented by HCDR1, HCDR2, and HCDR3, respectively, and three light chain complementarity determining regions represented by LCDR1, LCDR2, and LCDR3, respectively, wherein the antibody or the antigen-binding fragment thereof is selected from any one of the following:
A-I, wherein an amino acid sequence of the heavy chain complementarity determining region HCDR1 is as set forth in SEQ ID NO: 1, an amino acid sequence of the heavy chain complementarity determining region HCDR2 is as set forth in SEQ ID NO: 2, an amino acid sequence of the heavy chain complementarity determining region HCDR3 is as set forth in SEQ ID NO: 3, an amino acid sequence of the light chain complementarity determining region LCDR1 is as set forth in SEQ ID NO: 4, an amino acid sequence of the light chain complementarity determining region LCDR2 is as set forth in SEQ ID NO: 5, and an amino acid sequence of the light chain complementarity determining region LCDR3 is as set forth in SEQ ID NO: 6;
A-II, wherein an amino acid sequence of the heavy chain complementarity determining region HCDR1 is as set forth in SEQ ID NO: 1, an amino acid sequence of the heavy chain complementarity determining region HCDR2 is as set forth in SEQ ID NO: 2, an amino acid sequence of the heavy chain complementarity determining region HCDR3 is as set forth in SEQ ID NO: 3, an amino acid sequence of the light chain complementarity determining region LCDR1 is as set forth in SEQ ID NO: 4, an amino acid sequence of the light chain complementarity determining region LCDR2 is as set forth in SEQ ID NO: 5, and an amino acid sequence of the light chain complementarity determining region LCDR3 is as set forth in SEQ ID NO: 7;
A-III, wherein an amino acid sequence of the heavy chain complementarity determining region HCDR1 is as set forth in SEQ ID NO: 8, an amino acid sequence of the heavy chain complementarity determining region HCDR2 is as set forth in SEQ ID NO: 9, an amino acid sequence of the heavy chain complementarity determining region HCDR3 is as set forth in SEQ ID NO: 10, an amino acid sequence of the light chain complementarity determining region LCDR1 is as set forth in SEQ ID NO: 11, an amino acid sequence of the light chain complementarity determining region LCDR2 is as set forth in SEQ ID NO: 12, and an amino acid sequence of the light chain complementarity determining region LCDR3 is as set forth in SEQ ID NO: 13; and
A-IV, wherein an amino acid sequence of the heavy chain complementarity determining region HCDR1 is as set forth in SEQ ID NO: 1, an amino acid sequence of the heavy chain complementarity determining region HCDR2 is as set forth in SEQ ID NO: 14, an amino acid sequence of the heavy chain complementarity determining region HCDR3 is as set forth in SEQ ID NO: 15, an amino acid sequence of the light chain complementarity determining region LCDR1 is as set forth in SEQ ID NO: 4, an amino acid sequence of the light chain complementarity determining region LCDR2 is as set forth in SEQ ID NO: 5, and an amino acid sequence of the light chain complementarity determining region LCDR3 is as set forth in SEQ ID NO: 6.

2. The antibody binding to IL-11 or the antigen-binding fragment thereof according to claim 1, comprising a heavy chain variable region and a light chain variable region, wherein the antibody or the antigen-binding fragment thereof is selected from any one of the following:
MA-I, wherein an amino acid sequence of the heavy chain variable region is as set forth in SEQ ID NO: 16, and an amino acid sequence of the light chain variable region is as set forth in SEQ ID NO: 17;
MA-II, wherein an amino acid sequence of the heavy chain variable region is as set forth in SEQ ID NO: 16, and an amino acid sequence of the light chain variable region is as set forth in SEQ ID NO: 18;
MA-III, wherein an amino acid sequence of the heavy chain variable region is as set forth in SEQ ID NO: 19, and an amino acid sequence of the light chain variable region is as set forth in SEQ ID NO: 20; and
MA-IV, wherein an amino acid sequence of the heavy chain variable region is as set forth in SEQ ID NO: 21, and an amino acid sequence of the light chain variable region is as set forth in SEQ ID NO: 17.

3. The antibody binding to IL-11 or the antigen-binding fragment thereof according to claim 2, further comprising a heavy chain constant region and a light chain constant region, wherein an amino acid sequence of the heavy chain constant region is as set forth in one of SEQ ID NO: 23, SEQ ID NO: 24, SEQ ID NO: 25, or SEQ ID NO: 26; and an amino acid sequence of the light chain constant region is as set forth in SEQ ID NO: 22.

4. The antibody binding to IL-11 or the antigen-binding fragment thereof according to claim 2, wherein the antibody or the antigen-binding fragment thereof is a chimeric antibody molecule, and the chimeric antibody molecule further comprises a human antibody constant region.

5. The antibody binding to IL-11 or the antigen-binding fragment thereof according to claim 1, comprising a heavy chain variable region and a light chain variable region, wherein the antibody or the antigen-binding fragment thereof is selected from any one of the following:
HA-I-A, wherein an amino acid sequence of the heavy chain variable region is as set forth in SEQ ID NO: 31, and an amino acid sequence of the light chain variable region is as set forth in SEQ ID NO: 32;
HA-I-B, wherein an amino acid sequence of the heavy chain variable region is as set forth in SEQ ID NO: 33, and an amino acid sequence of the light chain variable region is as set forth in SEQ ID NO: 34;
HA-I-C, wherein an amino acid sequence of the heavy chain variable region is as set forth in SEQ ID NO: 31, and an amino acid sequence of the light chain variable region is as set forth in SEQ ID NO: 35;
HA-I-D, wherein an amino acid sequence of the heavy chain variable region is as set forth in SEQ ID NO: 36, and an amino acid sequence of the light chain variable region is as set forth in SEQ ID NO: 37;
HA-II-A, wherein an amino acid sequence of the heavy chain variable region is as set forth in SEQ ID NO: 31, and an amino acid sequence of the light chain variable region is as set forth in SEQ ID NO: 38;
HA-II-B, wherein an amino acid sequence of the heavy chain variable region is as set forth in SEQ ID NO: 31, and an amino acid sequence of the light chain variable region is as set forth in SEQ ID NO: 39;
HA-II-C, wherein an amino acid sequence of the heavy chain variable region is as set forth in SEQ ID NO: 33, and an amino acid sequence of the light chain variable region is as set forth in SEQ ID NO: 39; and
HA-II-D, wherein an amino acid sequence of the heavy chain variable region is as set forth in SEQ ID NO: 36, and an amino acid sequence of the light chain variable region is as set forth in SEQ ID NO: 40.

6. The antibody binding to IL-11 or the antigen-binding fragment thereof according to claim 5, wherein the antibody or the antigen-binding fragment thereof further comprises a human antibody constant region.

7. The antibody binding to IL-11 or the antigen-binding fragment thereof according to claim 4 or 6, wherein the human antibody constant region comprises a human antibody heavy chain constant region and a human antibody light chain constant region, and wherein an amino acid sequence of the human antibody heavy chain constant region is as set forth in one of SEQ ID NO: 27, SEQ ID NO: 28, or SEQ ID NO: 29; and an amino acid sequence of the human antibody light chain constant region is as set forth in SEQ ID NO: 30.

8. The antibody binding to IL-11 or the antigen-binding fragment thereof according to claim 1, wherein the antibody or the antigen-binding fragment thereof is one of Fab, F(ab')2, Fv or ScFv, or a combination thereof.

9. A nucleic acid molecule, wherein the nucleic acid molecule encodes the antibody binding to IL-11 or the antigen-binding fragment thereof according to claim 1.

10. A recombinant DNA expression vector, wherein the recombinant DNA expression vector comprises the nucleic acid molecule according to claim 9.

11. A host cell transfected with the recombinant DNA expression vector according to claim 10, wherein the host cell is a prokaryotic cell, a yeast cell, an insect cell, or a mammalian cell;
preferably, the host cell is a mammalian cell, and the mammalian cell is a HEK293 cell, a CHO cell, or an NS0 cell.

12. A drug, wherein the drug comprises the antibody binding to IL-11 or the antigen-binding fragment thereof according to claim 1.

13. A detection reagent, wherein the detection reagent is used to detect the antibody binding to IL-11 or the antigen-binding fragment thereof according to claim 1.

14. A combined drug composition, wherein the combined drug composition comprises the antibody binding to IL-11 or the antigen-binding fragment thereof according to claim 1, and further comprises a second composition selected from the group consisting of an antibody binding to PD-1, PD-L1, VEGF, VEGFR, TNF-α, or TSLP and an antigen-binding fragment thereof.

15. Use of the antibody binding to IL-11 or the antigen-binding fragment thereof according to claim 1 in the manufacture of a medicament for treating or preventing a fibrotic disease, inflammation, cancer, or an autoimmune disease in humans.

16. The use according to claim 15, wherein the fibrotic disease is selected from the group consisting of fibrosis of heart, liver, kidney, lung, gallbladder, bladder, stomach, bone marrow, penis, breast, blood vessel, eye, pancreas, spleen, brain, intestine, muscle, and skin.

17. The use according to claim 15, wherein the inflammation is selected from the group consisting of hepatitis, myocarditis, nephritis, pneumonia, cholecystitis, cystitis, gastritis, osteomyelitis, prostatitis, mastitis, pancreatitis, enteritis, arthritis, polymyositis, dermatomyositis, and dermatitis.

18. The use according to claim 15, wherein the cancer is selected from the group consisting of leukemia, lung cancer, gastric cancer, esophageal cancer, ovarian cancer, head and neck cancer, melanoma, renal cancer, breast cancer, colorectal cancer, liver cancer, pancreatic cancer, and bladder cancer.

19. The use according to claim 15, wherein the autoimmune disease is selected from the group consisting of psoriasis, Crohn's disease, primary biliary cirrhosis, systemic lupus erythematosus, and multiple sclerosis.
